# EUROPÄISCHE PATENTANMELDUNG

(11) **EP 1 444 982 A1**
(43) Veröffentlichungstag der Anmeldung: **11.08.2004**
(21) Anmeldenummer: 03002753.6
(22) Anmeldetag: 06.02.2003
(51) Int. Cl.: A61K 31/7076

(54) **Verwendung von Purinderivaten als selektive Kinase-Inhibitoren**

(71) Anmelder: MERCKLE GMBH, D-89079 Ulm (DE)
(72) Erfinder: Die Erfindernennung liegt noch nicht vor
(74) Vertreter: Riedl, Peter, Dr.

(57) **Zusammenfassung**

Die vorliegende Erfindung betrifft die Verwendung von Purinderivaten zur selektiven Inhibierung von Kinasen. Die Verbindungen sind daher zur Behandlung von Erkrankungen brauchbar, welche in Zusammenhang mit der Kinase-Aktivität stehen.

## Beschreibung

Die vorliegende Erfindung betrifft die Verwendung von Purinderivaten zur Herstellung pharmazeutischer Mittel zur selektiven Inhibierung der Aktivität von Kinasen.

Kinasen zählen zur Enzymgruppe der Phosphotransferasen, durch die Phosphatgruppen (meist von ATP) auf Hydroxylgruppen unter Ausbildung von Phosphatmonoestergruppen übertragen werden. Dadurch werden die betreffenden Verbindungen in der Regel in den aktivierten, d.h. in den zur Einschleusung in Stoffwechselreaktionen bereiten Zustand überführt. Kinasen sind somit an zahlreichen Stoffwechselreaktionen und an der Ausbildung bestimmter Krankheitsbilder beteiligt.

Kinasen und ihre Beteiligung an Stoffwechselvorgängen waren Gegenstand intensiver Untersuchungen, siehe beispielsweise *Progress in Biophysics & Molecular Biology* 71 (1999) 479-500, *BioEssays* Vol. 18 no. 7, 567-577 (1996), *Molecular Medicine Today,* October 1999 (Vol. 5), 439-447 und *Cell*. *Mol*. *Life Sci*. 55 (1999) 1230-1254.

Kinase-Inhibitoren sind bereits bekannt, beispielsweise Pyridinyl-imidazol-Verbindungen, siehe die erwähnte Publikation in *Molecular Medicine Today.* Aus medizinischer Sicht wäre es wünschenswert, Verbindungen zur Verfügung zu haben, welche selektiv die Aktivität bestimmter Kinasen inhibieren.

Überraschenderweise wurde nun gefunden, dass bestimmte Purinderivate selektive Kinase-Inhibitoren sind.

Die vorliegende Erfindung betrifft daher die Verwendung von Purinderivaten der Formel I 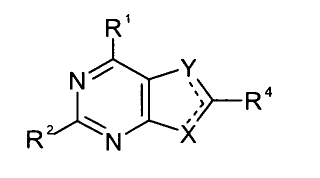 worin
R¹ für H, Phenylamino, Halogen-substituiertes Phenylamino, Phenoxy, Halogen-substituiertes Phenoxy, Benzylsulfanyl, am Phenylring Halogen-substituiertes Benzylsulfanyl, Phenylsulfanyl, Halogen-substituiertes Phenylsulfanyl, Phenyl, Halogen-substituiertes Phenyl, Styryl, am Phenylring Halogen-substituiertes Styryl, Benzyl, am Phenylring Halogen-substituiertes Benzyl, Amino, C₁-C₄-Alkoxyl, Halogen oder H steht;
R² für H oder C₁-C₄-Alkyl steht,
die gestrichelte Linie eine chemische Bindung zwischen X und CR⁴ oder zwischen Y und CR⁴ symbolisiert;
X für N oder NR³ steht und Y für N, CR⁵ oder NR⁶ steht, wobei X für NR³ und Y für N oder CR⁵ stehen, wenn die gestrichelte Linie eine Bindung zwischen Y und CR⁴ bedeutet oder X für N und Y für NR⁶ stehen, wenn die gestrichelte Linie eine Bindung zwischen X und CR⁴ bedeutet;
R³ für H, C₁-C₄-Alkyl, Benzyl, am Phenylring Halogen-substituiertes Benzyl steht, Phenyl oder Halogen-substituiertes Phenyl steht;
R⁴ für H, Halogen, Phenyl, das gegebenenfalls durch 1 oder 2 C₁-C₄-Alkyl-S(O)ₙsubstituiert ist, oder für -C≡C-(CH₂)ₘ-OH steht, wobei n für 0, 1 oder 2 steht und m für 1, 2 oder 3 steht;
R⁵ für H steht und
R⁶ für H, Phenyl, Halogen-substituiertes Phenyl, Benzyl oder am Phenylring Halogen-substituiertes Benzyl steht
oder eines physiologisch verträglichen Salzes dieser Verbindungen
zur Herstellung eines pharmazeutischen Mittels zur selektiven Inhibierung der Aktivität von Kinasen.
C₁-C₄-Alkyl steht für geradkettige oder verzweigte Alkylgruppen mit 1 bis 4 Kohlenstoffatomen, beispielsweise Methyl, Ethyl, n-Propyl, Isopropyl, n-Butyl, sek-Butyl und tert.-Butyl.

Halogen steht für F, Cl, Br und I, vorzugsweise für F, Cl und insbesondere für F. Wenn R⁴ für Halogen steht, sind Cl, Br, I und insbesondere Cl und Br bevorzugt.

Halogen-substituiertes Phenyl oder Halogen-substituierte Phenylgruppe bedeutet vorzugsweise eine Phenylgruppe, die durch ein oder zwei Halogenatome substituiert ist (dies gilt auch für die Phenylgruppe in Phenylamino, Phenylsulfanyl, Phenoxy etc.). Die Substituenten befinden sich insbesondere in den Positionen 2 und 4. Besonders bevorzugt sind Phenylgruppen mit einem Substituenten, der insbesondere in 4-Position steht. Ganz besonders bevorzugt ist dabei ein Fluor-Substituent.

Styryl steht für 2-Phenylvinyl, wobei sowohl die cis- als auch die trans-Form umfasst sind.

Wenn R⁴ für eine Phenylgruppe steht, die durch C₁-C₄-Alkyl-S(O)ₙ-substituiert ist, so stehen diese Gruppen vorzugsweise in 2- und/oder 4-Position. Besonders bevorzugt ist die Phenylgruppe mit einer Gruppe dieser Formel, insbesondere in 4-Position substituiert.

Bei den physiologisch verträglichen Salzen der Verbindungen der Formel I handelt es sich insbesondere um Säureadditionssalze. Die Säureadditionssalze werden gebildet mit anorganischen Säuren, wie Salzsäure, Schwefelsäure oder Phosphorsäure oder mit organischen Säuren, wie Weinsäure, Zitronensäure, Maleinsäure, Fumarsäure, Äpfelsäure, Mandelsäure, Ascorbinsäure, Gluconsäure und dergleichen.

Bei den erfindungsgemäßen Verbindungen kann zumindest folgendes tautomeres Gleichgewicht vorliegen (wenn X und Y für N bzw. NH stehen):

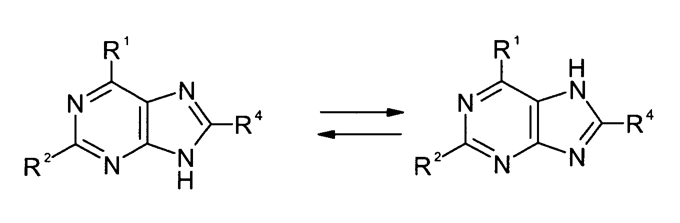

Die Erfindung umfasst auch die tautomeren Formen.

Bevorzugt ist die Verwendung von Verbindungen der Formel I, worin R¹ für Phenylamino, Halogen-substituiertes Phenylamino, Benzylsulfanyl, am Phenylring Halogen-substituiertes Benzylsulfanyl, Phenylsulfanyl, Halogen-substituiertes Phenylsulfanyl, Phenyl, Halogen-substituiertes Phenyl, Amino, C₁-C₄-Alkoxyl, Styryl oder am Phenylring Halogen-substituiertes Styryl steht.

Die Verbindungen der Formel I sind selektive Kinase-Inhibitoren. Vorzugsweise handelt es sich dabei um eine oder mehrere der folgenden Kinasen:

### c-Raf1

Diese Kinase ist eine ubiquitär im Cytosol lokalisierte Proteinkinase des Ras-Signalwegs, der für die Regulation des Zellwachstums entscheidend ist. C-Raf1 wird durch das Membran ständige Ras-Protein durch Phosphorylierung aktiviert. Die c-Rafl1 nachgeschaltete Signalübertragung läuft über die Proteinkinasen MEK1 und MEK2 (andere Bezeichnung: MKK1 und MKK2) zu den sogenannten Extracellular signal-regulated kinases ERK1 und ERK2. In Versuchen mit Zellkulturen und in Tiermodellen konnte gezeigt werden, dass durch Hemmung der c-Raf1-vermittelten Signalübertragung das Tumorwachstum gehemmt werden kann (*Bioorganic-and-Medicinal-Chemistry-Letters*. 2000 FEB 07; 10(3): 223-226 und *Proc. Amer. Assoc. Cancer Res.,* 2001, 42, 2921).

### GSK3β

Die Glycogen Synthase Kinase-3β (GSK3β) ist an vielen Signalübertragungswegen beteiligt. GSK3β wird durch Phosphorylierung aktiviert und aktiviert Transkriptionsfaktoren (activator protein-1, cAMP response element binding protein, NFkB). Hemmstoffe von GSK3β haben potenziell Nutzen zur Therapie von neurodegenerativen Krankheiten, Typ II-Diabetes, Schlaganfall, Krebs und chronisch entzündlichen Erkrankungen (*Medicinal-Research-Reviews,* 2002; 22(4): 373-384 und *Trends-in-Molecular-Medicine*, 2002; 8(3): 126-132).

### JNK3

Die cJun-N-terminal-Kinasen (auch: SAPK, stress activated protein kinases) aktivieren durch Phosphorylierung das c-Jun-Protein, das eine Erhöhung der Transkriptionsaktivität zahlreicher Gene auslöst, die Wachstum, Differenzierung oder Apoptose induzieren können. Hemmstoffe der JNK werden zur Behandlung von neurodegenerativen und chronisch entzündlichen Krankheiten in Betracht gezogen (*Drugs of the Future*, 2001, 26(19), 957-973).

### Lck

LSTRA cell kinase (Lck, auch: p56^{lck}) gehört zur Scr-Familie von Protein Tyrosin Kinasen und hat eine Schlüsselfunktion bei der T-Zell-Signalübertragungskaskade. Lck wird ausschließlich in Lymphozyten exprimiert. Hemmstoffe der Lck könnten zur Behandlung zahlreicher Krankheiten wie Rheumatoide Arthritis, Asthma, chronische entzündliche Darmerkrankung Psoriasis, Multiple Sklerose eingesetzt werden (*Current-Opinion-in-Investigational-Drugs*. 2001; 2(9): 1213-1219; Expert-Opinion-on-Therapeutic-Patents. 2001; 11 (2): 295-306; *Current-Topics-in-Microbiology-and-Immunology*. 1996; 205(-): 63-87 und *Expert-Opinion-on-Therapeutic-Patents.* 1995; 5(8): 805-817).

### ERK-1/-2, MEK1, SAPK2a

Die Proteinkinasen ERK-1 und -2 (**e**xtracellular **r**egulated **k**inase), die vorgeschaltete MAP Kinase MEK1 (auch: MKK1, MAPKK1) und SAPK2a (stress activated protein kinase 2a, auch: p38α MAP kinase) sind an Signalübertragungswegen beteiligt, die bei chronischen Entzündungen erhöhte Aktivität aufweisen. Als Folge dieser erhöhten Aktivität werden Zytokine wie der Tumor Nekrose Faktor α (TNFα) oder Interleukin 1β (IL-1β) vermehrt gebildet und ausgeschüttet, die den Entzündungsprozeß aufrecht erhalten. Die Hemmung dieser Kinasen durch Arzneistoffe führt zur Reduzierung pro-inflammatorischer Zytokine, wodurch der Entzündungsprozess abschwächt werden kann (*Trends Pharm. Sci*., 2002, 23(1 ), 40-45; *Mol*. *Med. Today,* 1999, 5(10), 439-447; *BioEssays*, 1996, 18(7), 567-577; *Gastroenterology,* 2002, 122, 7-14; und *Leukemia.* 2002 Apr; 16(4): 486-507

### Rho-kinase II (ROCK-II)

Es handelt sich um eine Serin/Threonin-Kinase, die an der Regulation der Kontraktion glatter Muskelzellen beteiligt ist und in Neuronen in der frühen Phase der Axonausbildung mitwirkt. Eine Wirkstoff-vermittelte Hemmung von Rock-II kann zur Behandlung erektiler Dysfunktion eingesetzt werden (*Drug-News-and-Perspectives* 2001; 14(10): 601-606).

### Protein-Kinase B (PKB)

Protein-Kinase B (PKB, andere Bezeichnung: Akt) ist an der Regulation intrazellulärer Prozesse beteiligt, die Proliferation oder Apoptose auslösen. Eine erhöhte Aktivität dieses Enzyms kann den Apoptoseprozess hemmen. In einigen humanen Krebsformen wurde eine erhöhte Expression von PKB nachgewiesen. Die therapeutische Anwendung von PKB-Inhibitoren erscheint daher bei Krebsformen angezeigt, die eine erhöhte PKB-Aktivität aufweisen. Die Hemmung der PKB-Aktivität könnten die apoptotischen Prozesse wieder initiieren. Auch in Kombination mit Chemotherapeutika sind Ansätze für eine therapeutische Verwendung gegeben. So wurde in experimentellen Modellen gezeigt, dass durch Wirkstoff-vermittelte Hemmung der PKB-Aktivität Tumorzellen (Nicht-kleinzelliges Lungenkarzinom, Pankreas-Karzinom) deutlich besser auf Zytostatika ansprechen (*Pharmacology-and-Therapeutics.* 2002; 93(2-3): 243-251; *Cancer-Res*. 2001 May 15; 61 (1 0): 3986-97; und *Clin-Cancer-Res*. 2001 Oct; 7(10): 3269-75).

### Protein-Kinase C (PKC)

Protein-Kinase C umfasst eine Gruppe von 12 Iso-Enzymen, die an zahlreichen intrazellulären Signalübertragungen beteiligt sind. Aufgrund der Schlüsselfunktion, die die PKC in verschiedenen Organfunktionen einnimmt und der Tatsache, dass die Aktivität bei bestimmten Krankheitsformen erhöht ist, macht die PKC zu einem attraktiven therapeutischen Target. Eine therapeutische Verwendung von PKC-Inhibitoren ist bei neuronalen Störungen, kardiovaskulären Krankheiten, der diabetischen Retinopathie oder bei verschiedenen Krebsformen möglich (*Am-J-Ophthalmol*. 2002 May; 133(5): 693-8; *Pharmacol-Res.* 2001 Nov; 44(5): 353-61; *Mol-Cell-Biochem*. 2001 Sep; 225(1-): 97-107; und *Curr-Drug-Targets*. 2000 Sep; 1(2): 163-83).

### Protein-Kinase A

Die Protein Kinase A (PKA) ist ein im inaktiven Zustand tetrameres Enzym, das durch Bindung des Botenstoffs cAMP in die aktive Form überführt wird. Aktivierte PKA induziert verschiedene zelluläre Signalübertragungskaskaden. Bei neoplastischen Transformationen und bei der Angiogenese hat die PKA zentrale Bedeutung. Die Hemmung der PKA wird als Möglichkeit zur Tumortherapie angesehen. Die Arzneistoff-vermittelte Hemmung der PKA-Hemmung erhöht die Sensitivität von Tumorzellen gegen Strahlen- und Chemotherapie. Daher ist die Hemmung der PKA insbesondere als unterstützende Maßnahme zur Behandlung verschiedener Krebsformen attraktiv (*Ann. NYAcad. Sci*., 2002, 968, 139-147; *Clin-Cancer-Res*. 2001; 7(8): 2537-44; *Ann. NY Acad*. *Sci*., 2002, 968, 158-72).

### Bevorzugte Ausführungsformen:

1) Zur Inhibierung von c-RAF verwendet man vorzugsweise Verbindungen der Formel I 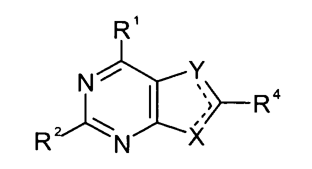 worin
   R¹ für Benzylsulfanyl, am Phenylring Halogen-substituiertes Benzylsulfanyl, Phenylamino, Halogen-substituiertes Phenylamino, Phenylsulfanyl, Halogen-substituiertes Phenylsulfanyl, Styryl, am Phenylring Halogen-substituiertes Styryl oder Amino steht;
   R² für H oder C₁-C₄-Alkyl steht;
   die gestrichelte Linie eine Bindung zwischen X und CR⁴ oder zwischen Y und CR⁴ bedeutet;
   X für N und Y für CR⁵ oder NR⁶ stehen, wenn die gestrichelte Linie eine Bindung zwischen X und CR⁴ bedeutet oder
   X für NR³ und Y für N stehen, wenn die gestrichelte Linie eine Bindung zwischen Y und CR⁴ bedeutet;
   R³ für H oder C₁-C₄-Alkyl, Benzyl oder am Phenylring Halogen-substituiertes Benzyl steht;
   R⁴ und R⁵ für H stehen; und
   R⁶ für H, Benzyl oder am Phenylring Halogen-substituiertes Benzyl steht.
   Besonders bevorzugt verwendet man dabei Verbindungen der Formel la 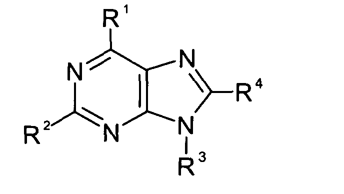 worin
   R¹ für Benzylsulfanyl, am Phenylring Halogen-substituiertes Benzylsulfanyl, Phenylamino, Halogen-substituiertes Phenylamino, Phenylsulfanyl, Halogen-substituiertes Phenylsulfanyl, Styryl, am Phenylring Halogen-substituiertes Styryl oder Amino steht;
   R² und R⁴ für H stehen;
   R³ für H, C₁-C₄-Alkyl, Benzyl oder am Phenylring Halogen-substituiertes Benzyl steht;
   oder Verbindungen der Formel Ib 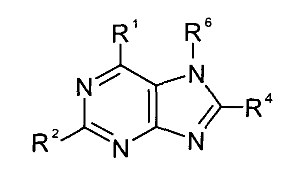 worin R¹ für Phenylsulfanyl, Halogen-substituiertes Phenylsulfanyl oder Amino steht; R² und R⁴ für H stehen;
   R⁶ für Benzyl oder am Phenylring Halogen-substituiertes Benzyl steht;
   oder Verbindungen der Formel Ic 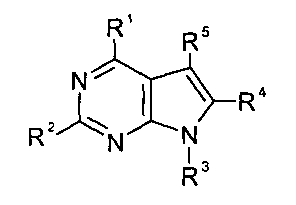 worin R¹ für Phenylsulfanyl oder Halogen-substituiertes Phenylsulfanyl steht und R², R³ und R⁵ für H stehen.
   Eine bevorzugte Ausführungsform der Erfindung ist somit die Verwendung dieser Verbindungen zur Inhibierung der Aktivität von c-RAF und insbesondere zur Behandlung von Erkrankungen, die durch die Aktivität von c-RAF bedingt sind, vorzugsweise zur Behandlung des Tumorwachstums.
2) Zur Inhibierung von GSK3β verwendet man vorzugsweise Verbindungen der Formel la, worin R¹ für Benzylsulfanyl, am Phenylring Halogen-substituiertes Benzylsulfanyl, Phenylamino, Halogen-substituiertes Phenylamino, Phenyl oder Halogen-substituiertes Phenyl steht,
   R² für H steht,
   R³ für H oder C₁-C₄-Alkyl steht, und
   R⁴ für H, Phenyl oder Halogen-substituiertes Phenyl steht.
   Eine bevorzugte Ausführungsform der Erfindung ist somit die Verwendung dieser Verbindungen zur Inhibierung der Aktivität von GSK3β, insbesondere zur Behandlung von Erkrankungen, die im Zusammenhang mit der Aktivität von GSK3β stehen, vorzugsweise zur Behandlung von neurodegenerativen Krankheiten, Diabetes mellitus, insbesondere Typ II, Schlaganfall, Krebs und chronisch entzündlichen Erkrankungen.
3) Die Inhibierung von JNK3 erfolgt vorzugsweise mit Verbindungen der Formel Id 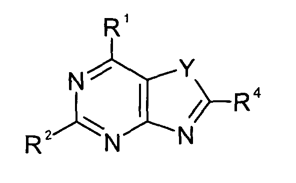 worin R¹ für C₁-C₄-Alkoxy oder Amino steht, R² und R⁴ für H stehen, Y für NR⁶ steht, wobei R⁶ für Benzyl oder am Phenylring Halogen-substituiertes Benzyl steht.
   Eine bevorzugte Ausführungsform der Erfindung ist somit die Verwendung dieser Verbindungen zur Inhibierung der Aktivität von JNK3, insbesondere zur Behandlung von Erkrankungen, die im Zusammenhang mit der Aktivität von JNK3 stehen, vorzugsweise zur Behandlung von neurodegenerativen und chronisch entzündlichen Erkrankungen.
4) Die Inhibierung von Lck erfolgt vorzugsweise mit Verbindungen der Formel le 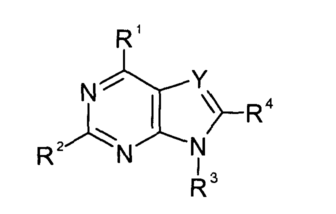 worin R¹ für Phenylamino, Halogen-substituiertes Phenylamino, Phenylsulfanyl, Halogen-substituiertes Phenylsulfanyl, Styryl oder am Phenylring Halogen-substituiertes Styryl steht,
   R² für H steht,
   R³ für H oder C₁-C₄-Alkyl steht,
   R⁴ für H oder Phenyl, das gegebenenfalls durch ein oder zwei C₁-C₄-Alkyl-S(O)ₙsubstituiert ist, steht, wobei n für 0, 1 oder 2 steht,
   Y für N oder CR⁵ steht und
   R⁵ für H steht.
   Vorzugsweise handelt es sich dabei um die Verbindungen der Formel la 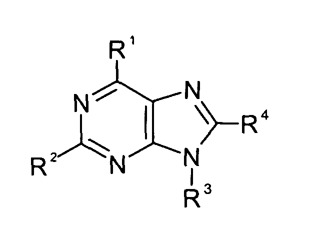 worin R¹ für Phenylamino, Halogen-substituiertes Phenylamino, Phenylsulfanyl, Halogen-substituiertes Phenylsulfanyl, Styryl oder am Phenylring Halogen-substituiertes Styryl steht,
   R² für H steht,
   R³ für H oder C₁-C₄-Alkyl steht,
   R⁴ für H oder Phenyl steht, das durch C₁-C₄-Alkyl-S(O)ₙ-substituiert ist, wobei n für 0,1 oder 2 steht
   oder
   um Verbindungen der Formel Ic 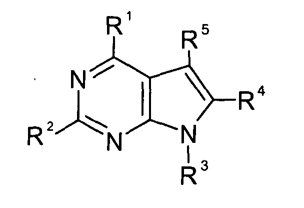 worin R¹ für Phenylsulfanyl oder Halogen-substituiertes Phenylsulfanyl steht, und R², R³, R⁴ und R⁵ für H stehen.
   Eine bevorzugte Ausführungsform der Erfindung ist somit die Verwendung dieser Verbindungen zur Herstellung eines pharmazeutischen Mittels zur Inhibierung der Aktivität von Lck, insbesondere zur Behandlung von Erkrankungen, die im Zusammenhang mit der Aktivität von Lck stehen, vorzugsweise zur Behandlung von rheumatoider Arthritis, Asthma, chronischer entzündlicher Darmerkrankung, Psoriasis oder Multipler Sklerose.
5) Zur Inhibierung von MAPK1 verwendet man vorzugsweise die Verbindungen der Formel la 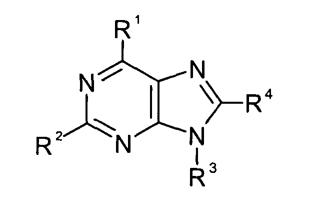 worin R¹ für Phenylamino, Halogen-substituiertes Phenylamino, Styryl oder Halogen-substituiertes Styryl steht,
   R² und R⁴ für H stehen,
   R³ für H oder C₁-C₄-Alkyl steht,
   oder Verbindungen der Formel Ib, worin R¹ für Amino steht, R² und R⁴ für H stehen und R⁶ für Benzyl oder am Phenylring Halogen-substituiertes Benzyl steht.
   Eine bevorzugte Ausführungsform der Erfindung ist somit die Verwendung dieser Verbindungen zur Herstellung eines pharmazeutischen Mittels zur Inhibierung der Aktivität von MAPK1, insbesondere zur Behandlung von Erkrankungen, die in Zusammenhang mit MAPK1 stehen, vorzugsweise zur Behandlung entzündlicher Erkrankungen.
6) Zur Inhibierung von MAPK2 verwendet man vorzugsweise die Verbindungen der Formel la 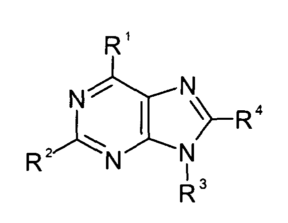 worin R¹ für Phenylamino oder Halogen-substituiertes Phenylamino steht,
   R² und R³ für H oder C₁-C₄-Alkyl stehen und
   R⁴ für H steht.
   Eine bevorzugte Ausführungsform der Erfindung ist somit die Verwendung dieser Verbindungen zur Herstellung eines pharmazeutischen Mittels zur Inhibierung der Aktivität von MAPK2, insbesondere zur Behandlung von Erkrankungen, die in Zusammenhang mit MAPK2 stehen, vorzugsweise zur Behandlung entzündlicher Erkrankungen.
7) Zur Inhibierung von MEK1 verwendet man vorzugsweise die Verbindungen der Formel le 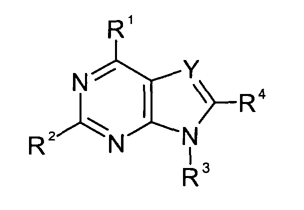 worin R¹ für Phenylamino, Halogen-substituiertes Phenylamino, Phenylsulfanyl oder Halogen-substituiertes Phenylsulfanyl steht,
   R², R³ und R⁴ für H stehen,
   Y für N oder CR⁵ steht und
   R⁵ für H steht.
   Eine bevorzugte Ausführungsform der Erfindung ist somit die Verwendung dieser Verbindungen zur Herstellung eines pharmazeutischen Mittels zur Inhibierung der Aktivität von MEK1, insbesondere zur Behandlung von Erkrankungen, die in Zusammenhang mit der Aktivität von MEK1 stehen, vorzugsweise zur Behandlung von entzündlichen Erkrankungen.
8) Zur Inhibierung von PKA verwendet man vorzugsweise Verbindungen der Formel la 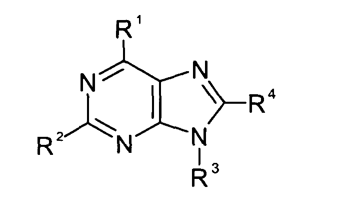 worin R¹ für Phenylamino oder Halogen-substituiertes Phenylamino steht und R², R³ und R⁴ für H stehen.
   Eine bevorzugte Ausführungsform der Erfindung ist somit die Verwendung dieser Verbindungen zur Herstellung eines pharmazeutischen Mittels zur Inhibierung von PKA, insbesondere zur Behandlung von Erkrankungen, die in Zusammenhang mit PKA stehen, vorzugsweise zur Behandlung von Krebs.
9) Zur Inhibierung von PKB verwendet man vorzugsweise die Verbindungen der Formel la 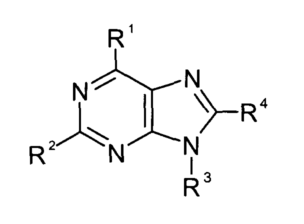 worin R¹ für Phenylsulfanyl, Halogen-substituiertes Phenylsulfanyl, Phenyl oder Halogen-substituiertes Phenyl steht,
   R² für H steht,
   R³ für H oder C₁-C₄-Alkyl steht,
   R⁴ für Phenyl oder -C≡C-(CH₂)ₘ-OH steht, wobei m für 1, 2 oder 3 steht.
   Eine bevorzugte Ausführungsform der Erfindung ist somit die Verwendung dieser Verbindungen zur Herstellung eines pharmazeutischen Mittels zur Inhibierung von PKB, insbesondere zur Behandlung von Erkrankungen, die in Zusammenhang mit der Aktivität von PKB stehen, vorzugsweise zur Behandlung von Krebs, insbesondere zur Behandlung von Krebsformen, die eine erhöhte PKB-Aktivität aufweisen.
10) Zur Inhibierung von ROCK-II verwendet man vorzugsweise Verbindungen der Formel le 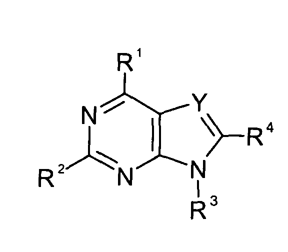 worin R¹ für Phenylamino, Halogen-substituiertes Phenylamino, Phenylsulfanyl oder Halogen-substituiertes Phenylsulfanyl steht,
   R² und R⁴ für H stehen,
   R³ für H oder C₁-C₄-Alkyl steht,
   Y für N oder CR⁵ steht und
   R⁵ für H steht.
   Eine bevorzugte Ausführungsform der Erfindung ist somit die Verwendung dieser Verbindungen zur Herstellung eines pharmazeutischen Mittels zur Inhibierung der Aktivität von ROKII, insbesondere zur Behandlung von Erkrankungen, die in Zusammenhang mit der Aktivität von ROCK-II stehen, vorzugsweise zur Behandlung erektiler Dysfunktion.
11) Zur Inhibierung von SAPK2a verwendet man vorzugsweise die Verbindungen der Formel le 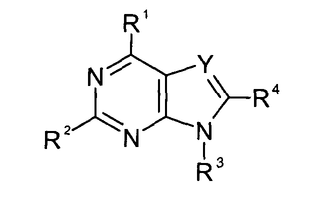 worin R¹ für Phenylamino, Halogen-substituiertes Phenylamino, Benzylsulfanyl, am Phenylring Halogen-substituiertes Benzylsulfanyl, Phenylsulfanyl oder Halogen-substituiertes Phenylsulfanyl steht,
   R² für H steht,
   R³ für H, C₁-C₄-Alkyl, Benzyl oder am Phenylring Halogen-substituiertes Benzyl steht,
   R⁴ für H, Phenyl oder Phenyl, das durch C₁-C₄-Alkyl-S(O)ₙ- substituiert ist, steht, wobei n für 0,1 oder 2 steht,
   Y für N oder CR⁵ steht und
   R⁵ für H steht.
   Eine bevorzugte Ausführungsform der Erfindung ist somit die Verwendung dieser Verbindungen zur Herstellung eines pharmazeutischen Mittels zur Inhibierung der Aktivität von SAPK2a, insbesondere zur Behandlung von Erkrankungen, die in Zusammenhang mit der Aktivität von SAPK2a stehen, vorzugsweise zur Behandlung entzündlicher Erkrankungen.

Die Erfindung betrifft auch die Verbindungen der Formel I, worin R¹ für Benzylsulfanyl, am Phenylring Halogen-substituiertes Benzylsulfanyl, Phenylsulfanyl oder Halogen-substituiertes Phenylsulfanyl steht,
R² für H oder C₁-C₄-Alkyl steht,
die gestrichelte Linie eine chemische Bindung zwischen X und CR⁴ oder zwischen Y und CR⁴ bedeutet,
X für N oder NR³ steht und Y für N, CR⁵ oder NR⁶ steht, wobei X für NR³ und Y für N oder CR⁵ steht, wenn die gestrichelte Linie eine Bindung zwischen Y und CR⁴ bedeutet oder X für N und Y für NR⁶ steht, wenn die gestrichelte Linie eine chemische Bindung zwischen X und CR⁴ bedeutet,
R³ für H, C₁-C₄-Alkyl, Benzyl oder am Phenylring Halogen-substituiertes Benzyl steht,
R⁴ für H, Phenyl oder Phenyl, das durch 1 oder 2 C₁-C₄-Alkyl-S(O)ₙ- substituiert ist, oder für -C=C-(CH₂)ₘ-OH steht,
n für 0, 1 oder 2 steht,
m für 1, 2 oder 3 steht,
R⁵ für H, und
R⁶ für H, Phenyl, Halogen-substituiertes Phenyl oder am Phenylring Halogen-substituiertes Benzyl steht,
und die physiologisch verträglichen Salze davon.

Ein Teil der Verbindungen der Formel I ist bekannt. Die Herstellung der bekannten und der neuen Verbindungen erfolgt nach bekannten Verfahren, sie wird im Folgenden beispielhaft erläutert:

Die Einführung der gegebenenfalls Halogen-substituierten Phenylamino, Phenoxy und Phenylsulfanylreste in 6-Position erfolgt durch Umsetzung von 6-Chlor-9H-purin mit dem entsprechenden Anilin, Phenol oder Thiophenol. Die Umsetzung wird in Anwesenheit einer Base, wie Triethylamin oder einem Alkalimetallhydroxid, in wässrigem oder wässrig-alkoholischem Medium durchgeführt.

Die Einführung der gegebenenfalls Halogen-substituierten Benzylsulfanyl-Verbindungen in 6-Position kann ausgehend von 6-Mercapto-9H-purin durch Umsetzung mit dem entsprechenden Benzylchlorid in Anwesenheit einer Base, wie einem Alkalimetallhydroxid, in wässrigem Medium erfolgen. Das 6-Mercapto-9Hpurin ist aus 6-Chlor-9H-purin durch Umsetzung mit Thioharnstoff erhältlich.

Die Einführung eines gegebenenfalls Halogen-substituierten Phenylrestes in 6-Position gelingt durch Umsetzung von 4,6-Dichlor-5-nitropyrimidin mit der entsprechenden Phenylboronsäure in Anwesenheit von katalytischen Mengen Pd(0)(PPh₃)₄ und einer Base, wie Natriumcarbonat. Aus dem erhaltenen Pyrimidinderivat wird durch Umsetzung mit einem C₁-C₄-Alkylamin das in 4-Position Alkylamino-substituierte Pyrimidinderivat erhalten. Reduktion der Nitrogruppe, beispielsweise mit Fe²⁺ und anschließende Umsetzung mit Orthoameisensäuretriethylester ergibt die entsprechende Verbindung der Formel I mit R⁴ = H.

Die Einführung der Aminogruppe in 6-Position erfolgt durch Umsetzung des entsprechenden 6-Chlorpurinderivates mit Ammoniak in alkoholischer Lösung.

Die Einführung der gegebenenfalls substituierten Phenyl- oder Styrylgruppe in 6-Position erfolgt durch Umsetzung des entsprechenden 6-Chlorpurinderivates mit der entsprechend substituierten Phenyl- oder Styrylboronsäure in Anwesenheit von Pd(0)(PPh₃)₄ in Anwesenheit einer Base wie Kaliumcarbonat. Alternativ kann die Styrylgruppe durch Umsetzung des entsprechenden 6-Chlorpurinderivates (dessen NH-Gruppe geschützt ist, z.B. mit der Tetrahydropyran-2-yl-gruppe) in einer Wittig-Reaktion mit Methyl-triphenylphosphoniumbromid und dem entsprechenden Benzaldehyd in 6-Position eingeführt werden. Umsetzung des 6-Chlorpurinderivates mit dem entsprechenden Benzyl-tributylphosphoniumbromid und n-Butyllithium ergibt die in 6-Position benzylsubstituierten Purinderivate.

Die Einführung von OC₁-C₄-Alkyl erfolgt durch Umsetzung des entsprechenden 6-Chlorpurinderivates mit ammoniakalischem C₁-C₄-Alkanol.

Die Verbindungen der Formel I, bei der X für NR³ und R³ für C₁-C₄-Alkyl stehen, werden ausgehend von 4,6-Dichlor-5-nitropyrimidin erhalten. Die Aminogruppe in 4-Position wird durch Umsetzung mit einem C₁-C₄-Alkylamin eingeführt, anschließend wird die Nitrogruppe zur Aminogruppe reduziert, beispielsweise mit Fe²⁺, und schießlich wird die erhaltene Verbindung mit Orthoameisensäuretriethylester in ethanolischer Lösung in Anwesenheit katalytischer Mengen einer Säure, beispielsweise p-Toluolsulfonsäure, zu dem in 9-Position alkylierten Purinderivat umgesetzt.

Die Alkylierung des Stickstoffatoms in 9-Position mit einer gegebenenfalls Halogen-substituierten Benzylgruppe erfolgt durch Umsetzung von 6-Chlor-9H-purin mit Benzylchlorid in Dimethylformamid in Anwesenheit einer Base, wie K₂CO₃.

Die Einführung eines Substituenten in 8-Position erfolgt durch Umsetzung von 6-Chlor-N'4'-methyl-pyrimidin-4,5-diamin (dessen Herstellung oben beschrieben ist) mit dem entsprechenden Orthocarbonsäureester, z.B. Orthobenzoesäuretriethylester, in Acetanhydrid. Alternativ kann ein Rest in 8-Position durch Umsetzung des entsprechenden 8-Brompurinderivates mit der entsprechenden Phenylboronsäure in Anwesenheit von Pd(0)(PPh₃)₄ und einer Base, wie Kaliumcarbonat, eingeführt werden. Alkinreste können in 8-Position durch Umsetzung des 8-Brompurins mit dem Alkin in Anwesenheit von Cu-I-Salzen und PCl₂(PPh₃)₂ eingeführt werden (Sonogashira-Reaktion; siehe *Palladium Reagents and Catalysts-Innovations in Organic Synthesis*, Wiley Inc., New York 1996).

Verbindungen der Formel I, worin Y für CR⁵ steht, werden durch Ringschluss von 6-Amino-5-(2,2-diethoxyethyl)pyrimidin-4-ol in saurer Lösung hergestellt. Das dabei anfallende 7H-Pyrrolo[2,3-d]pyrimidin-4-ol kann durch Umsetzung mit Phosphoroxichlorid in die entsprechende 4-Chlorverbindung überführt werden, was die anschließende Einführung weiterer Reste nach den oben beschriebenen Verfahren in 4-Position ermöglicht.

Die Verbindungen der Formel I, worin Y für NR⁵ steht, sind durch Umsetzung der am Aminstickstoff entsprechend substituierten Aminoacetonitrile mit Formamidinacetat in einem alkoholischen Lösungsmittel, wie Isopropanol, n-Butanol, Ethylenglykol etc., erhältlich.

Die Herstellung der Verbindungen der Formel I ist in den Beispielen näher erläutert. In den Beispielen nicht beschriebene Verbindungen können in analoger Weise erhalten werden.

Die erfindungsgemäßen Verbindungen können entweder als einzelne therapeutische Wirkstoffe oder als Mischungen mit anderen therapeutischen Wirkstoffen verabreicht werden. Die Verbindungen können als solche verabreicht werden, im Allgemeinen werden sie jedoch in Form pharmazeutischer Mittel dosiert und verabreicht, d. h. als Mischungen der Wirkstoffe mit geeigneten pharmazeutischen Trägern oder Verdünnungsmittel. Die Verbindungen oder Mittel können oral oder parenteral verabreicht werden, vorzugsweise werden sie in oralen Dosierungsformen gegeben.

Die Art des pharmazeutischen Mittels oder Trägers bzw. des Verdünnungsmittels hängt von der gewünschten Verabreichungsform ab. Orale Mittel können beispielsweise als Tabletten oder Kapseln vorliegen und können übliche Exzipienzien enthalten, wie Bindemittel (z. B. Sirup, Akazia, Gelatine, Sorbit, Tragant oder Polyvinylpyrrolidon), Füllstoffe (z. B. Lactose, Zucker, Maisstärke, Calciumphosphat, Sorbit oder Glycin), Gleitmittel (z. B. Magnesiumstearat, Talcum, Polyethylenglycol oder Siliciumdioxid), desintegrierende Mittel (z. B. Stärke) oder Netzmittel (z. B. Natriumlaurylsulfat). Flüssige Oralpräparate können in Form wässriger oder öliger Suspensionen, Lösungen, Emulsionen, Sirupe, Elixiere oder Sprays und dergleichen sein. Sie können auch als Trockenpulver vorliegen, das zur Rekonstitution mit Wasser oder einem anderen geeigneten Träger aufbereitet wird. Derartige flüssige Präparate können übliche Additive, beispielsweise Suspendiermittel, Geschmacksstoffe, Verdünnungsmittel oder Emulgatoren enthalten. Für die parenterale Verabreichung kann man Lösungen oder Suspensionen mit üblichen pharmazeutischen Trägern einsetzen.

Die erfindungsgemäßen Verbindungen oder Mittel können an Säuger (Mensch oder Tier) in einer Dosis von etwa 0,5 mg bis 100 mg pro kg Körpergewicht pro Tag verabreicht werden. Sie können in einer Einzeldosis oder in mehreren Dosen gegeben werden. Die Selektivität der Verbindungen als Kinase-Inhibitoren wurde anhand nachstehender Testsysteme untersucht.

### Kinaseverdünnung:

Alle Kinasen werden auf zehnfache Arbeitskonzentration vor Anwendung in dem Essay verdünnt. Die Zusammensetzung des Verdünnungspuffers ist für jede Kinase in der nachfolgenden Tabelle angegeben.

| **Pufferkomposition** | **Kinase(n)** |
|---|---|
| 50 mM Tris pH 7.5, 0.1 mM EGTA, 0.1 mM NaVanadate, 0.1% β-mercaptoethanol, 1 mg/ml BSA. | c-RAF, MAPK1, MAPK2, SAPK2a, |
| 50 mM Tris pH 7.5, 0.1 mM EGTA, 0.1% β-mercaptoethanol, 1 mg/ml BSA. | JNK3 ROCK-II |
| 25 mM Tris pH 7.5, 0.1 mM EGTA, 0.1% β-mercaptoethanol, 1 mg/ml BSA. | MEK1. |
| 20 mM MOPS pH 7.0, 1 mM EDTA, 0.1% β-mercaptoethanol, 0.01% Brij-35, 5% glycerol, 1 mg/ml BSA. | GSK3β, Lck, PKA, PKBα, PKBβ, |

### Substrate:

Alle Substrate werden gelöst und auf eine Stammlösung in entsalztem Wasser verdünnt.

### GSK3β (h):

GSK3β (h)(5-10mU) in einem Endreaktionsvolumen von 25 µl wird mit 8 mM MOPS pH 7.0, 0.2 mM EDTA, 20 µM YRRAAVPPSPSLSRHSSPHQS(p) EDEEE (phospho GS2 Peptid), 10 mM MgAcetat und [γ-³³P-ATP] inkubiert (spezifische Aktivität etwa 500 cpm/pmol, Konzentration wie erforderlich). Die Reaktion wird durch Zugabe von Mg²⁺[γ-³³P-ATP] initiiert. Nach 40-minütiger Inkubation bei Raumtemperatur wird die Reaktion durch Zugabe von 5 µl einer 3 %igen Phosphorsäurelösung gestoppt. 10 µl der Reaktion werden dann auf ein P30-Filter gegeben und dreimal jeweils 5 Minuten in 50 mM Phosphorsäure und einmal in Methanol gewaschen. Anschließend wird getrocknet und eine Szintillationszählung durchgeführt.

### Lck (h):

Lck (h)(5-10 mU) in einem Endreaktionsvolumen von 25 µl wird mit 50 mM Tris pH 7.5, 0.1 mM EGTA, 0.1 mM NaVanadate, 250 µM KVEKIGEGTYGVVYK (Cdc2 Peptid), 10 mM MgAcetat und [γ-³³P-ATP] inkubiert (spezifische Aktivität etwa 500 cpm/pmol, Konzentration wie erforderlich). Die Reaktion wird durch Zugabe von Mg²⁺[γ-³³P-ATP] initiiert. Nach 40-minütiger Inkubation bei Raumtemperatur wird die Reaktion durch Zugabe von 5 µl einer 3 %igen Phosphorsäurelösung gestoppt. 10 µl der Reaktion werden dann auf ein P30-Filter gegeben und dreimal jeweils 5 Minuten in 50 mM Phosphorsäure und einmal in Methanol gewaschen. Anschließend wird getrocknet und eine Szintillationszählung durchgeführt.

### SAPK2a (h):

SAPK2a (h) wird in einem Endreaktionsvolumen von 25 µl mit 25 mM Tris pH 7,5, 0.02 mM EGTA, 0.33 mg/ml Myelin-Basisprotein, 10 mM MgAcetat und [γ-³³P-ATP] inkubiert (spezifische Aktivität etwa 50 cpm/pmol, Konzentration wie erforderlich). Die Reaktion wird durch Zugabe von Mg²⁺[γ-³³P-ATP] initiiert. Nach 40-minütiger Inkubation bei Raumtemperatur wird die Reaktion durch Zugabe von 5 µl einer 3 %igen Phosphorsäurelösung gestoppt. 10 µl der Reaktion werden dann auf ein P30-Filter gegeben und dreimal jeweils 5 Minuten in 50 mM Phosphorsäure und einmal in Methanol gewaschen. Anschließend wird getrocknet und eine Szintillationszählung durchgeführt.

### PKBα (h):

PKBα (h)(5-10mU) wird in einem Endreaktionsvolumen von 25 µl mit 8 mM MOPS pH 7.0, 0.2 mM EDTRA, 30 µM GRPRTSSFAEGKK, 10 mM MgAcetat und [γ-³³P-ATP] inkubiert (spezifische Aktivität etwa 50 cpm/pmol, Konzentration wie erforderlich). Die Reaktion wird durch Zugabe von Mg²⁺[γ-³³P-ATP] initiiert. Nach 40-minütiger Inkubation bei Raumtemperatur wird die Reaktion durch Zugabe von 5 µl einer 3 %igen Phosphorsäurelösung gestoppt. 10 µl der Reaktion werden dann auf ein P30-Filter gegeben und dreimal jeweils 5 Minuten in 50 mM Phosphorsäure und einmal in Methanol gewaschen. Anschließend wird getrocknet und eine Szintillationszählung durchgeführt.

### ROCK-II(r):

ROCK-II(r)(5-10mU) in einem Endreaktionsvolumen von 25 µl wird mit 50 mM Tris pH 7.5, 0.1 mM EGTA, 30 µM KEAKEKROQEQIAKRRRLSSLRA STSKSGGSQK, 10 mM MgAcetat und [γ-³³P-ATP] inkubiert (spezifische Aktivität etwa 50 cpm/pmol, Konzentration wie erforderlich). Die Reaktion wird durch Zugabe von Mg²⁺[γ-³³P-ATP] initiiert. Nach 40-minütiger Inkubation bei Raumtemperatur wird die Reaktion durch Zugabe von 5 µl einer 3 %igen Phosphorsäurelösung gestoppt. 10 µl der Reaktion werden dann auf ein P30-Filter gegeben und dreimal jeweils 5 Minuten in 50 mM Phosphorsäure und einmal in Methanol gewaschen. Anschließend wird getrocknet und eine Szintillationszählung durchgeführt.

### PKA (b):

PKA (b)(5-10 mU) in einem Endreaktionsvolumen von 25 µl wird mit 8 mM MOPS pH 7.0, 0.2 mM EDTA, 30 µM LRRASLG (Kemptite), 10 mM MgAcetat und [γ-³³P-ATP] inkubiert (spezifische Aktivität etwa 50 cpm/pmol, Konzentration wie erforderlich). Die Reaktion wird durch Zugabe von Mg²⁺[γ-³³P-ATP] initiiert. Nach 40-minütiger Inkubation bei Raumtemperatur wird die Reaktion durch Zugabe von 5 µl einer 3 %igen Phosphorsäurelösung gestoppt. 10 µl der Reaktion werden dann auf ein P30-Filter gegeben und dreimal jeweils 5 Minuten in 50 mM Phosphorsäure und einmal in Methanol gewaschen. Anschließend wird getrocknet und eine Szintillationszählung durchgeführt.

### c-RAF (ha):

c-RAF (ha)(5-10 mU) in einem Endreaktionsvolumen von 25 µl wird mit 25 mM Tris pH 7.5,.0.02 mM EGTA, 0.66 mg/ml Myelin-Basisprotein, 10 mM MgAcetat und [γ-³³P-ATP] inkubiert (spezifische Aktivität etwa 50 cpm/pmol, Konzentration wie erforderlich). Die Reaktion wird durch Zugabe von Mg²⁺[γ-³³P-ATP] initiiert. Nach 40-minütiger Inkubation bei Raumtemperatur wird die Reaktion durch Zugabe von 5 µl einer 3 %igen Phosphorsäurelösung gestoppt. 10 µl der Reaktion werden dann auf ein P30-Filter gegeben und dreimal jeweils 5 Minuten in 50 mM Phosphorsäure und einmal in Methanol gewaschen. Anschließend wird getrocknet und eine Szintillationszählung durchgeführt.

### MEK1 (h):

MEK1 (h)(1-5 mU) in einem Endreaktionsvolumen von 25 µl wird mit 50 mM Tris pH 7.5, 0.2 mM EGTA, 0.1 % b-Mercaptoethanol, 0,01 % Brij-35, 1 µM inaktives MAPK2 (m), 10 mM MgAcetat und kaltem ATP inkubiert (Konzentration wie erforderlich). Die Reaktion wird durch Zugabe von MgATB initiiert. Nach 40-minütiger Inkubation bei Raumtemperatur werden 5 ml dieses Inkubationsgemisches dazu verwendet, den oben beschriebenen MAPK2-Assay zu initiieren.

Die erhaltenen Ergebnisse sind in der nachfolgenden Tabelle zusammengestellt:

**Tabelle:**

| Hemmung von Kinasen in % bei 30µM Testsubstanz (100 µM ATP) | | | | | | | | | | | |
|---|---|---|---|---|---|---|---|---|---|---|---|
| Verbd. Nr. | c-RAF (h) | GSK3β (h) | JNK3 (r) | Lck (h) | MAPK1 (h) | MAPK2 (h) | MEK1 (h) | PKA (h) | PKB (h) | ROCKII (h) | SAPK2a (h) |
| 1 | 19 | 13 | 11 | 39 | 22 | 33 | 19 | 32 | 14 | 48 | 34 |
| 2 | 24 | 13 | 10 | 42 | 33 | 48 | 34 | 40 | 18 | 52 | 30 |
| 13 | 44 | 26 | 5 | 18 | 16 | 42 | 4 | 0 | 0 | 0 | 0 |
| 9 | 53 | 35 | 7 | 22 | 15 | 12 | 7 | 13 | 4 | 28 | 50 |
| 10 | 47 | 0 | 12 | 16 | 9 | 5 | 0 | 12 | 6 | 18 | 29 |
| 14 | 27 | 14 | 19 | 9 | 36 | 57 | 8 | 0 | 0 | 7 | 13 |
| 15 | 6 | 1 | 0 | 5 | 3 | 13 | 0 | 8 | 0 | 33 | 54 |
| 16 | 29 | 0 | 8 | 13 | 2 | 12 | 2 | 10 | 0 | 37 | 72 |
| 18 | 12 | 0 | 9 | 2 | 9 | 12 | 6 | 1 | 0 | 6 | 38 |
| 23 | 12 | 0 | 12 | 5 | 10 | 0 | 4 | 3 | 42 | 1 | 44 |
| 24 | 14 | 22 | 16 | 20 | 11 | 1 | 7 | 3 | 29 | 9 | 29 |
| 41 | 24 | 26 | 12 | 17 | 20 | 1 | 5 | 0 | 31 | 0 | 23 |
| 43 | 28 | 0 | 25 | 0 | 26 | 5 | 8 | 7 | 0 | 6 | 8 |
| 44 | 0 | 5 | 30 | 0 | 19 | 3 | 3 | 7 | 0 | 1 | 8 |
| 42a | 25 | 7 | 17 | 19 | 17 | 11 | 7 | 1 | 0 | 5 | 7 |
| 52 | 22 | 11 | 0 | 56 | 11 | 4 | 41 | 0 | 0 | 36 | 56 |
| 53 | 43 | 4 | 5 | 52 | 10 | 2 | 36 | 0 | 0 | 42 | 79 |
| 59 | 0 | 4 | 6 | 30 | 0 | 0 | 3 | 0 | 4 | 1 | 27 |
| 60 | 4 | 4 | 10 | 30 | 0 | 0 | 1 | 4 | 10 | 0 | 44 |
| 62 | 10 | 0 | 7 | 16 | 9 | 3 | 4 | 11 | 14 | 5 | 36 |
| 72 | 66 | 20 | 2 | 37 | 50 | 20 | 7 | 4 | 7 | 22 | 17 |
| 74 | 0 | 1 | 0 | 0 | 0 | 0 | 6 | 0 | 1 | 1 | 64 |
| 75 | 0 | 0 | 0 | 16 | 0 | 0 | 4 | 0 | 1 | 5 | 64 |
| 76 | 0 | 0 | 0 | 12 | 17 | 0 | 1 | 0 | 13 | 2 | 67 |
| 77 | 0 | 8 | 0 | 16 | 6 | 0 | 3 | 0 | 0 | 0 | 27 |
| 79 | 0 | 0 | 0 | 11 | 0 | 0 | 0 | 0 | 33 | 0 | 3 |

Als selektiv wird eine Verbindung betrachtet, die eine Hemmung von wenigstens 25 %, vorzugsweise wenigstens 30 % gegenüber einer der getesteten Kinasen aufweist.

Die nachfolgenden Beispiele erläutern die Erfindung, ohne sie zu begrenzen

### Verwendete Abkürzungen:

- DMF: Dimethylformamid
- EtOAc: Ethylacetat
- EtOH: Ethanol
- THF: Tetrahydrofuran
- DHK: Dreihalskolben
- DME: Dimethoxyethan
- n-BuLi: n-Butyllithium

### Phenyl-(9H-purin-6-yl)-amin (1)

Eine Lösung von 6-Chlor-9H-purin (0.62 g, 4 mmol), Anilin (0.74 g, 8 mmol) und Triethylamin (0.81 g, 8 mmol) in 10 mL n-Butanol wird 4.5 h unter Rückfluss gerührt. Beim Abkühlen bildet sich ein hellgrüner Niederschlag, der abfiltriert und mit Methanol und Diethylether gewaschen wird. Die Titelverbindung wird unter Vakuum getrocknet.
- C₁₁H₉N₅ (Mᵣ 211.23): 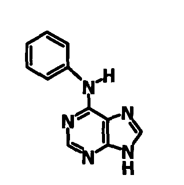
- Ausbeute: 642 mg (76%)
- Schmelzpunkt: 278 °C
- IR (ATR): 1613, 1578, 1502, 1479, 1443, 1362, 1302, 1250, 931, 751, 693, 669 cm⁻¹
- ¹H-NMR (DMSO-*d*_{*6*}): δ (ppm) 6.98-7.09 (m, 1H, Phenyl), 7.28-7.41 (m, 2H, Phenyl), 7.95-7.99 (m, 2H, Phenyl), 8.28 (s, 1H, C²-H), 8.37 (s, 2H, C⁸-H), 9.78 (s, 1H, N-H), 13.18 (s, 1H, N-H)

### (4-Fluoro-phenyl)-(9H-purin-6-yl)-amin (2).

Eine Lösung von 6-Chlor-9H-purin (0.62 g, 4 mmol), 4-Fluoranilin (0.89 g, 8 mmol) und Triethylamin (0.81 g, 8 mmol) in 10 mL n-Butanol wird 4.5 h unter Rückfluss gerührt. Beim Abkühlen bildet sich ein hellgrüner Niederschlag, der abfiltriert und mit Methanol und Diethylether gewaschen wird. Die Titelverbindung wird unter Vakuum getrocknet.
- C₁₁H₈FN₅ (Mᵣ 229.22): 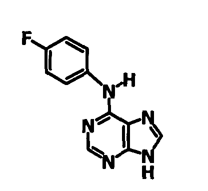
- Ausbeute: 730 mg (80%)
- Schmelzpunkt: 338 °C
- IR (ATR): 1637, 1579, 1513, 1479, 1338, 1254, 1206, 1159, 1087, 929, 829, 794, 672 cm⁻¹
- ¹H-NMR (CDCl₃): δ (ppm) 7.13-7.22 (m, 2H, 4-F-Phenyl), 7.94-8.01 (m, 2H, 4-F-Phenyl), 8.28 (s, 1H, C²-H), 8.36 (s, 2H, C⁸-H), 9.87 (s, 1H, N-H), 13.19 (s, 1H, N-H)

### 6-Phenoxy-9H-purin (3).

Eine Schmelze aus 6-Chlor-9H-purin (0.5 g, 3.25 mmol) und 4-Fluorphenol (1.1 g, 9.75 mmol) wird 4.5 h bei 110 °C gerührt. Auf die erkaltete Schmelze gibt man ca. 4 mL Methanol und lässt über Nacht im Kühlschrank stehen. Die feinen Kristalle werden abgesaugt und mit Diethylether gewaschen. Das Rohprodukt wird aus Isopropanol umkristallisiert. Die Titelverbindung wird unter Vakuum getrocknet.
- C₁₁H₈N₄O (Mᵣ 212.21): 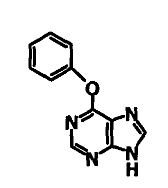
- Schmelzpunkt: 217 °C
- IR (ATR): 1615, 1593, 1578, 1480, 1404, 1339, 1302, 1197, 964, 875, 780, 689 cm⁻¹
- ¹H-NMR (CDCl₃): δ (ppm) 7.26-7.52 (m, 5H, Phenyl), 8.41 (s, 1H, C²-H), 8.48 (s, 2H, C⁸-H), 13.60 (s, 1H, N-H)

### 6-(4-Fluoro-phenoxy)-9H-purin (4).

Eine Schmelze aus 6-Chlor-9H-purin (0.5 g, 3.25 mmol) und Phenol (1.2 g, 12.77 mmol) wird 4.5 h bei 110 °C gerührt Auf die erkaltete Schmelze gibt man ca. 4 mL Methanol und lässt über Nacht im Kühlschrank stehen. Die feinen Kristalle werden abgesaugt und mit Diethylether gewaschen. Das Rohprodukt wird aus Isopropanol umkristallisiert. Die Titelverbindung wird unter Vakuum getrocknet.
- C₁₁H₇FN₄O (Mᵣ 230.20): 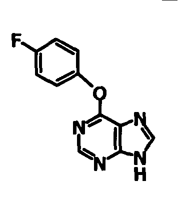
- Schmelzpunkt: 218°C
- IR (ATR): 1634, 1621, 1502, 1445, 1400, 1309, 1235, 1186, 1078, 959, 878, 828 cm⁻¹
- ¹H-NMR (CDCl₃): δ (ppm) 7.26-7.40 (m, 4H, 4-F-Phenyl), 8.43 (s, 1H, C²-H), 8.53 (s, 2H, C⁸-H), 13.60 (s, 1H, N-H)

### Phenylamino-acetonitril (5).

Eine Lösung von Anilin (9.30 g, 0.1 mol), Chloracetonitril (7.55 g, 0.1 mol), und Triethylamin (10.1 g, 0.1 mol) in 40 mL Ethanol wird unter Zusatz von KBr (0.5 g, 4.2 mmol) 4 h unter Rückfluss gerührt. Das Lösungsmittel wird abrotiert und der Rückstand in Ethylacetat aufgenommen und geschüttelt. Die Suspension wird abfiltriert. Das Filtrat wird zweimal mit Wasser gewaschen, über Na₂SO₄ getrocknet und einrotiert. Der ölige Rückstand wird im Vakuum destilliert (162°C-164°C, 12 mbar). Die Titelverbindung fällt als gelbes Öl an, das im Kühlschrank kristallisiert.
- C₈H₈N₂ (Mᵣ 132.17): 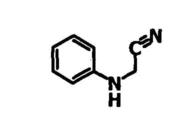
- Ausbeute: 2.23 g (17%)

### (4-Fluoro-phenylamino)-acetonitril (6).

Eine Lösung von 4-Fluoranilin (28.86 g, 0.26 mol), Chloracetonitril (19.71 g, 0.26 mol), und Triethylamin (26.36 g, 0.26 mol) in 90 mL Ethanol wird unter Zusatz von KBr (1 g, 8.4 mmol) 4 h unter Rückfluss gerührt. Das Lösungsmittel wird abrotiert und der Rückstand in Ethylacetat aufgenommen und geschüttelt. Die Suspension wird abfiltriert. Das Filtrat wird zweimal mit Wasser gewaschen, über Na₂SO₄ getrocknet und einrotiert. Der ölige Rückstand wird im Vakuum destilliert (161°C-169°C, 12 mbar). Die Titelverbindung fällt als gelbes Öl an, das im Kühlschrank kristallisiert.
- C₈H₇FN₂ (Mᵣ 150.16): 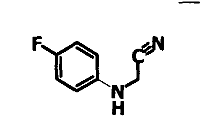
- Ausbeute: 22.03 g (57 %)

### 7-Phenyl-7H-purin (7)

Eine Lösung von Phenylamino-acetonitril (**5**) (1.65 g, 12.5 mmol) und Formamidinacetat (6.24 g, 60 mmol) in 13 mL n-Butanol wird 5 h unter Rückfluss gerührt. Das Lösungsmittel wird weitgehend abrotiert, der Rückstand in Wasser (150 mL) aufgenommen, mit Dichlormethan (3 × 200 mL) extrahiert, über Na₂SO₄ getrocknet und einrotiert. Der braune Rückstand wird säulenchromatographisch gereinigt (SiO₂ 60, CH₂Cl₂/EtOH 9+1). Die entsprechenden Fraktionen werden nicht bis zur Trockne einrotiert, sondern die ausgefallene weiße Titelverbindung wird abgesaugt und mit Ether gewaschen.
- C₁₁H₈N₄ (Mᵣ 196.21): 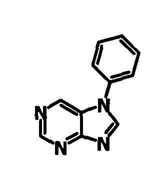
- Schmelzpunkt: 186 °C
- IR (ATR): 3052, 1597, 1500, 1467, 1413, 1351, 1310, 1200, 1154, 907, 799, 757, 696 cm⁻¹
- ¹H-NMR (DMSO-*d*_{*6*}): δ (ppm) 7.48-7.83 (m, 5H, Phenyl), 9.06 (s, 1H, C⁸H), 9.10 (s, 1H, C²-H), 9.22 (s, 1H, C⁶-H)
- ¹³C-NMR (DMSO-*d*_{*6*}): δ (ppm)

### 7-(4-Fluoro-phenyl)-7H-purin (8)

Eine Lösung von (4-Fluoro-phenylamino)-acetonitril (**6**). (3 g, 20 mmol) und Formamidin-acetat (15.61 g, 150 mmol) in 20 mL n-Butanol wird 5 h unter Rückfluss gerührt. Das Lösungsmittel wird weitgehend abrotiert, der Rückstand in Wasser (200 mL) aufgenommen, mit Dichlormethan (3 × 250 mL) extrahiert, über Na₂SO₄ getrocknet und einrotiert. Der braune Rückstand wird säulenchromatographisch gereinigt (SiO₂ 60, CH₂Cl₂/EtOH 9+1). Die entsprechenden Fraktionen werden nicht bis zur Trockne einrotiert, sondern die ausgefallene weiße Titelverbindung wird abgesaugt und mit Ether gewaschen.
- C₈H₉ClO₂S (Mᵣ 204.68): 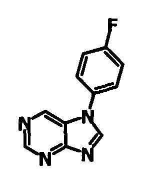
- Schmelzpunkt: 232 °C
- IR (ATR): 3073, 1604, 1592, 1507, 1416, 1317, 1216 (C-F), 1195, 1154, 908, 845, 817, 800, 695 cm⁻¹
- ¹H-NMR (DMSO-*d*_{*6*}): δ (ppm) 7.47-7.56 (m, 2H, 4-F-Phenyl), 7.83-7.92 (m, 2H, 4-F-Phenyl), 9.08 (s, 1H, C⁸-H), 9.20 (s, 2H, C²-H, C⁶-H)

### 6-Benzylsulfanyl-9H-purin (9)

6-Mercaptopurin (0.61 g, 3.6 mmol) und NaOH (0.4 g, 10 mmol) werden in 10 mL Wasser gelöst. Unter Rühren wird Benzylchlorid (0.5 g, 3,96 mmol) langsam zugetropft und 2.5 h weitergerührt. Mit Eisessig wird auf pH 5 eingestellt. Die ausgefallenen weißen Kristalle werden abgesaugt, mit Wasser gewaschen und an der Luft getrocknet. Nach säulenchromatographischer Reinigung (SiO₂ 60, CH₂Cl₂/EtOH 9+1) erhält man die Titel-verbindung als weiße Substanz.
- C₁₂H₁₀N₄S (Mᵣ 242.30): 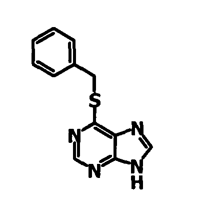
- Ausbeute: 0.64 g (73 %)
- Schmelzpunkt: 195 °C
- IR (ATR): 2555, 1569, 1421, 1381, 1326, 1232, 1133, 949, 926, 894, 836, 711, 693 cm⁻¹
- ¹H-NMR (DMSO-*d*_{*6*}): δ (ppm) 4.66 (s, 2H, CH₂), 7.24-7.50 (m, 5H, Phenyl), 8.47 (s, 1H, C⁸-H), 8.75 (s, 2H, C²-H), 13.52 (s, 1H, N⁹-H, austausch-bar)

### 6-(4-Fluoro-benzylsulfanyl)-9H-purin (10)

6-Mercaptopurin (0.61 g, 3.6 mmol) und NaOH (0.4 g, 10 mmol) werden in 10 mL Wasser gelöst. Unter Rühren wird 4-Fluorbenzylchlorid (0.57 g, 3,96 mmol) langsam zugetropft und anschließend 2.5 h weitergerührt. Mit Eisessig wird auf pH 5 eingestellt. Die ausgefallenen weißen Kristalle werden abgesaugt, mit Wasser gewaschen und an der Luft getrocknet. Nach säulenchromatographischer Reinigung (SiO₂ 60, CH₂Cl₂/EtOH 9+1) erhält man die Titelverbindung als weiße Substanz.
- C₁₂H₉FN4S (Mᵣ 260.29): 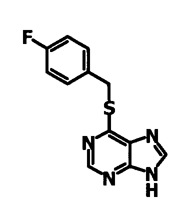
- Ausbeute: 0.25 g (27 %)
- Schmelzpunkt: 228 °C
- IR (ATR): 2924, 2854, 1728, 1567, 1506, 1391, 1324, 1233, 1216(C-F), 996, 919, 851, 808, 731 cm⁻¹
- ¹H-NMR (DMSO-*d*_{*6*}): δ (ppm) 4.65 (s, 2H, CH₂), 7.09-7.20 (m, 2H, 4-F-Phenyl), 7.46-7.55 (m, 2H, 4-F-Phenyl), 8.46 (s, 1H, C⁸-H), 8.74 (s, 2H, C²-H), 13.55 (s, 1H, N⁹-H, austauschbar)

### (6-Chloro-5-nitro-pyrimidin-4-yl)-methyl-amin (10a)

15 mL Eisessig werden unter Rühren langsam zu einer eisgekühlten Methylaminlösung (40% in Wasser) getropft. Die erhaltene Mischung wird während 1 h zu einer auf 4°C temperierten Lösung von 4,6-Dichlor-5-nitro-pyrimidin (11 g, 57 mmol) in 23 mL Dioxan zugetropft. Es wird 30 min. nachgerührt und anschließend werden unter starkem Rühren während 2 h 150 mL Eiswasser zugetropft. Die Temperatur wird auf 4-5 °C gehalten. Die ausgefallene Titelverbindung wird abgesaugt und zuerst mit Eiswasser, dann mit Hexan gewaschen.
- C₅H₅ClN₄O₂ (Mᵣ 188.57): 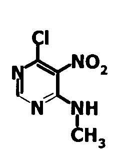
- Ausbeute: 8.7 g (81 %)

### 6-Chloro-N'4'-methyl-pyrimidin-4,5-diamin (11)

Eine Lösung von FeSO₄ x 7 H₂O (114.5 g, 0.41 mol) in 1375 mL Wasser wird auf 75 °C erhitzt. Unter starkem Rühren wird Ba(OH)₂ x 8 H₂O (134.6 g, 0.43 mol) portionsweise eingetragen und die entstehende Suspension auf 90 °C erhitzt und anschließend 20 min weitergerührt. (6-Chloro-5-nitro-pyrimidin-4-yl)-methyl-amin (**10a**) (8.7 g, 0.046 mol) wird portionsweise eingetragen und die sich schwarz färbende Suspension bei 90 °C 20 min nachgerührt. Die Suspension wird heiß filtriert und mit 375 mL kochendem Wasser nachgewaschen. Das Filtrat wird über Nacht im Kühlschrank stehen gelassen. Die Titelverbindung kristallisiert in langen Nadeln. Sie wird abgesaugt, mit 100 mL Wasser und 100 mL Petrolether gewaschen und unter Vakuum getrocknet.
- C₅H₇CIN₄ (Mᵣ 158.59): 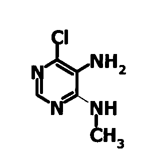
- Ausbeute: 3.9 g (54%)
- Schmelzpunkt: 162 °C

### 6-Chloro-9-methyl-9H-purin (12)

Eine Lösung von 6-Chloro-N'4'-methyl-pyrimidin-4,5-diamin (**11**) (5 g, 31.5 mmol) in einem Gemisch aus 50 mL Orthoameisensäuretriethylester und 25 mL Ethanol wird mit einer Spatelspitze p-Toluolsulfonsäuremonohydrat versetzt und 5 h refluxiert. Das Lösungsmittel wird abrotiert und der Rückstand aus Ethanol umkristallisiert. Die Titelverbindung wird abgesaugt und mit Petrolether gewaschen.
- C₆H₅CIN₄ (Mᵣ 168.59): 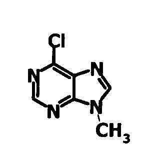
- Ausbeute: 0.19 g (31 %)
- Schmelzpunkt: 163 °C

### (9-Methyl-9H-purin-6-yl)-phenyl-amin (13)

Eine Lösung von 6-Chloro-9-methyl-9H-purin (**12**) (0.43 g, 2.6 mmol), Anilin (0.9 g, 8.1 mmol) und Triethylamin (0.78 g, 7.7 mmol) in 10 mL n-Butanol wird 8 h unter Rückfluss gerührt. Das Lösungsmittel wird abrotiert. Der Rückstand wird in einen Büchnertrichter überführt und mit wenig Ether und Hexan gewaschen. Nach säulenchromatographischer Reinigung (SiO₂ 60, CH₂Cl₂/EtOH 9+1) erhält man die Titelverbindung als weiße Substanz und trocknet sie unter Vakuum.
- C₁₂H₁₁N₅ (Mᵣ 225.25): 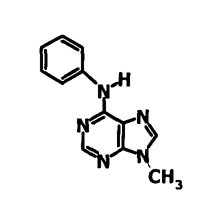
- Ausbeute: 430 mg (73%)
- Schmelzpunkt: 169°C
- IR (ATR): 3052, 1626, 1577, 1498, 1475, 1438, 1373, 1321, 1298, 1233, 1038, 737, 665 cm⁻¹
- ¹H-NMR (DMSO-*d*_{*6*}): δ (ppm) 3.80 (s, 3H, N⁹-CH₃), 6.99-7.37 (m, 3H, Phenyl), 7.96-7.99 (m, 2H, Phenyl), 8.28 (s, 1H, C²-H), 8.42 (s, 1H, C⁸-H), 9.84 (s, 1H, N-H, austauschbar)
- ¹³C-NMR (DMSO-*d*_{*6*}): 36.2, 121.7, 122.0, 123.5, 128.4, 138.9, 143.3, 150.5, 150.9, 153.4

### (4-Fluoro-phenyl)-(9-methyl-9H-purin-6-yl)-amin (14)

Eine Lösung von 6-Chloro-9-methyl-9H-purin (**12**) (0.43 g, 2.6 mmol), 4-Fluoranilin (0.58 g, 6.2 mmol) und Triethylamin (0.58 g, 5.7 mmol) in 10 mL n-Butanol wird 7 h unter Rückfluss gerührt. Das Lösungsmittel wird abrotiert. Der Rückstand wird in einen Büchnertrichter überführt und mit wenig Ether und Hexan gewaschen. Nach säulenchromatographischer Reinigung (SiO₂ 60, CH₂Cl₂/EtOH 9+1) und Trocknung unter Vakuum erhält man die Titelverbindung als weiße Substanz.
- C₁₂H₁₀FN₅ (Mᵣ 243.25): 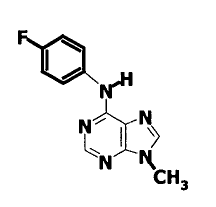
- Schmelzpunkt: 182°C
- IR (ATR): 1630, 1594, 1582, 1505, 1478, 1464, 1409, 1325, 1299, 1227 (C-F), 1197, 1024, 830,732, 658 cm⁻¹

### 6-Phenylsulfanyl-9H-purin (15)

Eine Lösung von 6-Chlor-9H-purin (0.5 g, 3.25 mmol), Thiophenol (1 g, 9.1 mmol) und NaOH (0.56 g, 14 mmol) in 7 mL Wasser wird 6 h bei 60 °C gerührt. Mit Eisessig wird auf pH 5 eingestellt. Die ausgefallenen weißen Kristalle werden abgesaugt, mit Wasser und wenig Diethylether gewaschen und an der Luft getrocknet. Nach säulenchromatographischer Reinigung (SiO₂ 60, CH₂Cl₂/EtOH 9+1) und Trocknung unter Vakuum erhält man die Titelverbindung als weiße Substanz.
- C₁₁H₈N₄S (Mᵣ 228.28): 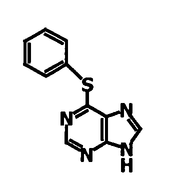
- Ausbeute: 290 mg (39%)
- Schmelzpunkt: 245 °C
- IR (ATR): 3063, 2960, 2800, 1590, 1560, 1433, 1384, 1320, 1234, 994, 921, 855, 749, 686 cm⁻¹
- ¹H-NMR (DMSO-*d*_{*6*}): 8 (ppm) 7.48-7.68 (m, 5H, Phenyl), 8.53 (s, 1H, C⁸-H), 8.56 (s, 1H, C²-H), 13.62 (s, 1H, N-H, austauschbar)

### 6-(4-Fluoro-phenylsulfanyl)-9H-purin (16)

Eine Lösung von 6-Chlor-9H-purin (0.5 g, 3.25 mmol), 4-Fluorthiophenol (1.2 g, 9.4 mmol) und NaOH (0.84 g, 21 mmol) in 10.5 mL Wasser wird 6 h bei 60 °C gerührt. Mit Eisessig wird auf pH 5 eingestellt. Die ausgefallenen weißen Kristalle werden abgesaugt, mit Wasser und wenig Diethylether gewaschen und an der Luft getrocknet. Nach säulenchromatographischer Reinigung (SiO₂ 60, CH₂Cl₂/EtOH 9+1) und Trocknung unter Vakuum erhält man die Titelverbindung als weiße Substanz.
- C₁₁H₇FN₄S (Mᵣ 246.27): 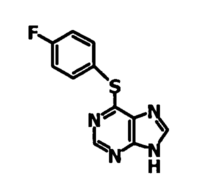
- Schmelzpunkt: 233 °C
- IR (ATR): 3056, 2943, 2792, 2680, 2563, 1591, 1565, 1493, 1379, 1323, 1230 (C-F), 1164, 946, 825, 816 cm⁻¹
- ¹H-NMR (DMSO-*d*_{*6*}): δ (ppm) 7.29-7.41 (m, 2H, 4-F-Phenyl), 7.64-7.74 (m, 2H, 4-F-Phenyl), 8.51 (s, 1H, C⁸-H), 8.55 (s, 1H, C²-H), 13.61 (s, 1H, N-H, austauschbar)

### 9-Methyl-6-phenylsulfanyl-9H-purin (17)

Eine Lösung von 6-Chloro-9-methyl-9H-purin (**12**) (0.43 g, 2.6 mmol), Thiophenol (0.68 g, 6.2 mmol) und Triethylamin (0.71 g, 7 mmol) in 10 mL n-Butanol wird 4.5 h unter Rückfluss gerührt. Das Lösungsmittel wird abrotiert. Der Rückstand wird in einen Büchnertrichter überführt und mit wenig Ether und Hexan gewaschen. Nach säulenchromatographischer Reinigung (SiO₂ 60, CH₂Cl₂/EtOH 9+1) und Trocknung unter Vakuum erhält man die Titelverbindung als weiße Substanz.
- C₁₂H₁₀N₄S (Mᵣ 242.30): 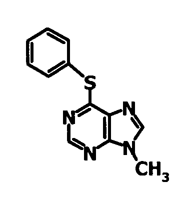
- Schmelzpunkt: 136 °C
- IR (ATR): 1561, 1435, 1323, 1238, 1222, 1167, 1141, 933, 855, 753, 746, 731, 683 cm⁻¹
- ¹H-NMR (DMSO-*d*_{*6*}): δ (ppm) 3.83 (s, 3H, N⁹-CH₃), 7.48-7.66 (m, 5H, Phenyl), 8.48 (s, 1H, C⁸-H), 8.57 (s, 1H, C²-H)
- ¹³C-NMR (DMSO-*d*_{*6*}): δ (ppm) 29.6 (N⁹-CH₃), 126.8, 129.2 (2C, Phenyl), 129.3 (C⁴', Phenyl), 129.8, 135.3 (2C, Phenyl), 145.6 (C⁸), 149.1, 151.4 (C²), 158.1

### 6-(4-Fluoro-phenylsulfanyl)-9-methyl-9H-purin (18)

Eine Lösung von 6-Chloro-9-methyl-9H-purin (**12**) (0.43 g, 2.6 mmol), 4-Fluorthiophenol (0.8 g, 6.2 mmol) und Triethylamin (0.71 g, 7 mmol) in 10 mL n-Butanol wird 5 h unter Rückfluss gerührt. Das Lösungsmittel wird abrotiert. Der Rückstand wird in einen Büchnertrichter überführt und mit wenig Ether und Hexan gewaschen. Nach säulenchromatographischer Reinigung (SiO₂ 60, CH₂Cl₂/EtOH 9+1) und Trocknung unter Vakuum erhält man die Titelverbindung als weiße Substanz.
- C₁₂H₉FN₄S (Mᵣ 260.29): 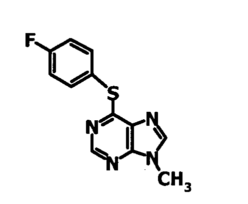
- Schmelzpunkt: 182°C
- Ausbeute: 350 mg (52%)
- IR (ATR): 1588, 1561, 1489, 1393, 1327, 1223, 1157, 1144, 933, 853, 832, 732 cm⁻¹
- ¹H-NMR (DMSO-*d*_{*6*}): δ (ppm) 3.83 (s, 3H, N⁹-CH₃), 7.31-7.40 (m, 2H, 4-F-Phenyl), 7.65-7.72 (m, 2H, 4-F-Phenyl), 8.48 (s, 1H, C⁸-H), 8.58 (s, 1H, C²-H)

### 9-Methyl-9H-purin-6-thiol (19)

Eine Lösung von 6-Chloro-9-methyl-9H-purin (**12**) (0.5 g, 3 mmol) und Thioharnstoff (0.58 g, 7.6 mmol) in 15 mL absolutiertem Ethanol wird 3 h unter Rückfluss gerührt. Während dieser Zeit fällt die Titelverbindung aus. Sie wird abgesaugt und mit Ethanol, Wasser und Diethylether gewaschen.
- C₆H₆N₄S₂ (Mᵣ 166.21): 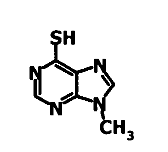
- Ausbeute: 470 mg (94%)
- ¹H-NMR (DMSO-*d*_{*6*}): δ (ppm) 3.74 (s, 3H, N⁹-CH₃), 8.18 (s, 1H, C⁸-H), 8.23 (s, 1H, C²-H), 13.70 (s, 1H, S-H, austauschbar)

### 6-Benzylsulfanyl-9-methyl-9H-purin (20)

9-Methyl-9H-purin-6-thiol (**19**). (0.47 g, 2.8 mmol) und NaOH (0.4 g, 10 mmol) werden in 10 mL Wasser gelöst. Unter Rühren wird Benzylchlorid (0.38 g, 3 mmol) langsam zugetropft und anschließend 2.5 h weitergerührt. Mit Eisessig wird auf pH 5 eingestellt. Die ausgefallenen weißen Kristalle werden abgesaugt, mit Wasser gewaschen und an der Luft getrocknet. Nach säulenchromatographischer Reinigung (SiO₂ 60, CH₂Cl₂/EtOH 95+5) erhält man die Titelverbindung als weiße Substanz.
- C₁₃H₁₂N₄S (Mᵣ 256.33): 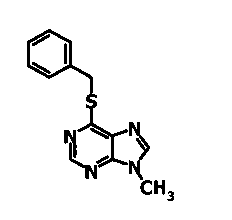
- Ausbeute: 430 mg (56%)
- Schmelzpunkt: 133 °C
- IR (ATR): 1567, 1493, 1416, 1390, 1322, 1233, 1171, 1142, 1066, 1047, 935, 860, 703 cm⁻¹
- ¹H-NMR (DMSO-*d*_{*6*}): δ (ppm) 3.82 (s, 3H, N⁹-CH₃), 4.66 (s, 2H, CH₂), 7.24-7.49 (m, 5H, Phenyl), 8.42 (s, 1H, C⁸-H), 8.77 (s, 2H, C²-H)
- ¹³C-NMR (DMSO-*d*_{*6*}): 29.6 (N⁹-CH₃), 31.5 (CH₂), 127.0 (C^{4'}, Phenyl), 128.3 (2C, Phenyl), 128.8 (2C, Phenyl), 130.2, 137.7 145.2 (C⁸), 148.8, 151.2 (C²), 158.3

### 6-(4-Fluoro-benzylsulfanyl)-9-methyl-9H-purin (21)

9-Methyl-9H-purin-6-thiol (**19**). (0.47 g, 2.8 mmol) und NaOH (0.4 g, 10 mmol) werden in 10 mL Wasser gelöst. Unter Rühren wird 4-Fluorbenzylchlorid (0.43 g, 3 mmol) langsam zugetropft und anschließend 2.5 h weitergerührt. Mit Eisessig wird auf pH 5 eingestellt. Die ausgefallenen weißen Kristalle werden abgesaugt, mit Wasser gewaschen und an der Luft getrocknet. Nach säulenchromatographischer Reinigung (SiO₂ 60, CH₂Cl₂/EtOH 9.5+0.5) erhält man die Titelverbindung als weiße Substanz.
- C₁₃H₁₁FN₄S (Mᵣ 274.32): 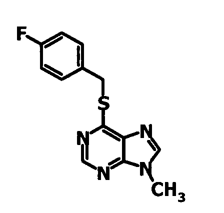
- Ausbeute: 450 mg (58%)
- Schmelzpunkt: 152 °C
- IR (ATR): 1578, 1563, 1505, 1416, 1328, 1235, 1222, 1156, 933, 860, 839, 728 cm⁻¹
- ¹H-NMR (DMSO-*d*_{*6*}): δ (ppm) 3.82 (s, 3H, N⁹-CH₃), 4.65 (s, 2H, CH₂), 7.09-7.18 (m, 2H, 4-F-Phenyl), 7.47-7.54 (m, 2H, 4-F-Phenyl), 8.43 (s, 1H, C⁸-H), 8.77 (s, 2H, C²-H)

### 6-Chloro-9-methyl-8-phenyl-9H-purin (22)

Eine Lösung von 6-Chloro-N'4'-methyl-pyrimidin-4,5-diamin (**11**) (2 g, 12.6 mmol) und Orthobenzoesäuretriethylester (7.44 g, 33.2 mmol) in Acetanhydrid wird 3 h refluxiert. Das Lösungsmittel wird abrotiert. Ein braunes Öl bleibt zurück. Nach säulenchromatographischer Reinigung (SiO₂ 60, CH₂Cl₂/EtOH 9.75+0.25) erhält man die Titelverbindung als weiße Substanz.
- C₁₂H₉ClN₄ (Mᵣ 244.69): 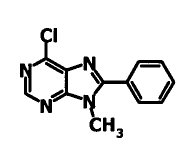
- Ausbeute: 530 mg (17%)
- Schmelzpunkt: °C
- IR (ATR): cm⁻¹
- ¹H-NMR (DMSO-*d*_{*6*}): δ (ppm)

### 9-Methyl-8-phenyl-6-phenylsulfanyl-9H-purin (23)

Eine Lösung von 6-Chloro-9-methyl-8-phenyl-9H-purin (**22**) (0.35 g, 1.43 mmol), Thiophenol (0.38 g, 6.2 mmol) und Triethylamin (0.39 g, 3.86 mmol) in 10 mL n-Butanol wird 4.5 h unter Rückfluss gerührt. Das Lösungsmittel wird abrotiert. Der Rückstand wird in einen Büchnertrichter überführt und mit wenig Ether und Hexan gewaschen. Nach säulenchromatographischer Reinigung (SiO₂ 60, CH₂Cl₂/EtOH 9.75+0.25) und Trocknung unter Vakuum erhält man die Titelverbindung als weiße Substanz.
- C₁₈H₁₄N₄S (Mᵣ 318.40): 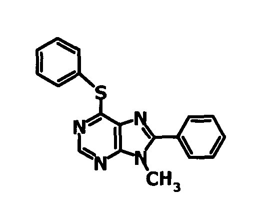
- Ausbeute: 200 mg (44%)
- Schmelzpunkt: 147 °C
- IR (ATR): 1557, 1475, 1440, 1331, 1247, 1152, 1022, 948, 863, 780, 745, 715, 702, 687 cm⁻¹
- ¹H-NMR (DMSO-*d*_{*6*}): δ (ppm) 3.90 (s, 3H, N⁹-CH₃), 7.50-7.64 (m, 8H, Phenyl), 7.92-7.97 (m, 2H, Phenyl), 8.60 (s, 1H, C²-H)
- ¹³C-NMR (DMSO-*d*_{*6*}): δ (ppm) 30.7 (N⁹-CH₃), 126.8, 128.7 (2C, Phenyl), 128.8, 129.2 (4C, Phenyl), 129.3 (C⁴', Phenyl), 129.8, 130.5 (C⁴", Phenyl), 135.3 (2C, Phenyl), 150.7, 151.2 (C²), 153.7, 157.5

### 6-(4-Fluoro-phenylsulfanyl)-9-methyl-8-phenyl-9H-purin (24)

Eine Lösung von 6-Chloro-9-methyl-8-phenyl-9H-purin (**22**) (0.30 g, 1.23 mmol), 4-Fluorthiophenol (0.4 g, 3 mmol) und Triethylamin (0.4 g, 4 mmol) in 10 mL n-Butanol wird 4.5 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, CH₂Cl₂/EtOH 9.75+0.25) und Trocknung unter Vakuum erhält man die Titelverbindung als weiße Substanz.
- C₁₈H₁₃FN₄S (Mᵣ 336.39): 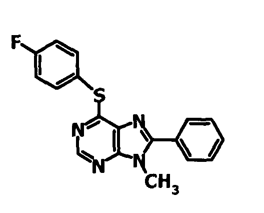
- Ausbeute: 310 mg (75%)
- Schmelzpunkt: 157 °C
- IR (ATR): 1588, 1561, 1488, 1331, 1248, 1221 (C-F), 1156, 1025, 947, 862, 819, 775, 731, 697 cm⁻¹
- ¹H-NMR (DMSO-*d*_{*6*}): δ (ppm) 3.90 (s, 3H, N⁹-CH₃), 7.32-7.40 (m, 2H, Phenyl), 7.61-7.74 (m, 5H, Phenyl), 7.92-7.97 (m, 2H, Phenyl), 8.60 (s, 1H, C²-H)

### Benzylamino-acetonitril (25)

In einer Lösung von Chloracetonitril (3.4 g, 45 mmol) und Benzylamin (3.21 g, 30 mmol) in 100 mL Acetonitril wird K₂CO₃ (8.3 g, 60 mmol) suspendiert. Es wird 16 h bei 60 °C gerührt. Das K₂CO₃ wird abfiltriert und das Filtrat einrotiert Der ölige Rückstand wird im Vakuum destilliert (157°C, 19 mbar). Die Titelverbindung fällt als farbloses Öl an.
- C₉H₁₀N₂ (Mᵣ 146.19): 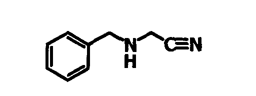
- Ausbeute: 2,15 g (49%)
- ¹H-NMR (CDCl₃): δ (ppm) 3.84 (s, 2H, CH₂), 3.89 (s, 2H, CH₂), 7.23-7.34 (m, 5H, Phenyl)

### (4-Fluoro-benzylamino)-acetonitril (26)

In einer Lösung von Chloracetonitril (3.76 g, 50 mmol) und 4-Fluorbenzylamin (4.16 g, 33 mmol) in 100 mL Acetonitril wird K₂CO₃ (8.3 g, 60 mmol) suspendiert. Es wird 16 h bei 60 °C gerührt. Das K₂CO₃ wird abfiltriert und das Filtrat einrotiert Der ölige Rückstand wird im Vakuum destilliert (152°C, 10 mbar). Die Titelverbindung fällt als farbloses Öl an.
- C₉H₉FN₂ (Mᵣ 164.18): 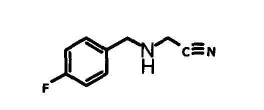
- Ausbeute: 2.56 g (47%)
- IR (ATR): cm⁻¹

### 7-Benzyl-7H-purin (27)

Eine Lösung von Benzylamino-acetonitril (**25**) (2.08 g, 14.2 mmol) und Formamidinacetat (4.44 g, 43 mmol) in 7 mL Ethylenglykol wird 4 h bei 140°C gerührt. Der abgekühlte Ansatz wird in 50 mL Wasser aufgenommen und dreimal mit je 50 mL Dichlormethan extrahiert. Die vereinigten Dichlormethanphasen werden über Na₂SO₄ getrocknet und einrotiert. Der braune, ölige Rückstand wird säulenchromatographisch gereinigt (SiO₂ 60, CH₂Cl₂/EtOH 9+1).
- C₁₂H₁₀N₄ (Mᵣ 210.24): 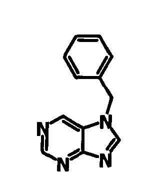
- Ausbeute: 300 mg (10%)
- Schmelzpunkt: 146 °C
- IR (ATR): 1600, 1484, 1447, 1409, 1377, 1271, 1171, 902, 896, 801, 784, 729, 695 cm⁻¹
- ¹H-NMR (DMSO-*d*_{*6*}): δ (ppm) 5.65 (s, 2H, CH₂), 7.29-7.47 (m, 5H, Phenyl), 8.94 (s, 1H, C⁸-H), 8.99 (s, 1H, C²), 9.14 (s, 1H, C⁶-H)
- ¹³C-NMR (DMSO-*d*_{*6*}): δ (ppm) 48.6 (CH₂), 124.8, 127.7 (2C, Phenyl), 128.1 (C⁴', Phenyl), 128.8 (2C, Phenyl), 135.8, 141.0 (C⁶), 149.3 (C⁸), 152.2 (C²), 160.1

### 7-(4-Fluoro-benzyl)-7H-purin (28)

Eine Lösung von (4-Fluoro-benzylamino)-acetonitril (**26**) (2.5 g, 15 mmol) und Formamidinacetat (4.44 g, 43 mmol) in 10 mL Ethylenglykol wird 4 h bei 140°C gerührt. Der abgekühlte Ansatz wird in 50 mL Wasser aufgenommen und dreimal mit je 50 mL Dichlormethan extrahiert. Die vereinigten Dichlormethanphasen werden über Na₂SO₄ getrocknet und einrotiert. Der braune, ölige Rückstand wird säulenchromatographisch gereinigt (SiO₂ 60, CH₂Cl₂/EtOH 9+1).
- C₁₂H₉FN₄ (Mᵣ 228.23): 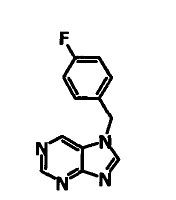
- Ausbeute: g (%)
- Schmelzpunkt: 138°C
- IR (ATR): 1606, 1511, 1480, 1410, 1375, 1343, 1305, 1269, 1218 (C-F), 1177, 1072, 903, 833, 803, 773 cm⁻¹
- ¹H-NMR (DMSO-*d*_{*6*}): δ (ppm) 5.61 (s, 2H, CH₂), 7.17-7.26 (m, 2H, 4-F-Phenyl), 7.49-7.56 (m, 2H, 4-F-Phenyl), 8.91 (s, 1H, C⁸-H), 8.98 (s, 1H, C²), 9.15 (s, 1H, C⁶-H)
- ¹³C-NMR (DMSO-*d*_{*6*}): δ (ppm)

### 9-Benzyl-6-chloro-9H-purin (29) und 7-Benzyl-6-chloro-7H-purin (30)

6-Chlor-9H-purin (1.55 g, 15 mmol) wird in 50 mL DMF gerührt, bis die Substanz vollständig gelöst ist. Man suspendiert K₂CO₃ (4.15 g, 30 mmol) und rührt 20 min. Benzylchlorid (1.89 g, 15 mmol) wird langsam zugegeben und es wird 22 h bei 20 °C gerührt. Das Lösungsmittel wird abrotiert und der braune, ölige Rückstand wird säulenchromatographisch getrennt (SiO₂ 60, EtOAc/Hexan 670+330). Zuerst eluiert 9-Benzyl6-chloro-9H-purin und danach 7-Benzyl-6-chloro-7H-purin.
- C₁₂H₉ClN₄ (Mᵣ 244.69): 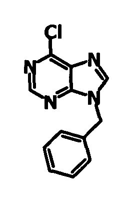 9-Benzyl-6-chloro-9H-purin
- Ausbeute: 1.11 g (46%)
- Schmelzpunkt: 88 °C
- IR (ATR): 3063, 1595, 1556, 1495, 1445, 1395, 1343, 1243, 1209, 1176, 951, 936, 856, 722, 698 cm⁻¹
- ¹H-NMR (DMSO-*d*_{*6*}): δ (ppm) 5.56 (s, 2H, CH₂), 7.28-7.38 (m, 5H, Phenyl), 8.81 (s, 1H, C⁸-H), 8.88 (s, 1H, C²-H)
- ¹³C-NMR (DMSO-*d*_{*6*}): δ (ppm) 48.9 (CH₂), 127.6 (2C, Phenyl), 127.9 (C⁴', Phenyl), 128.7 (2C, Phenyl), 135.9, 147.4 (C⁸), 149.1, 151.6 (C²), 151.7

- C₁₂H₉ClN₄ (Mᵣ 244.69): 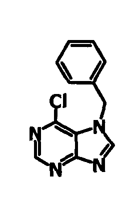 7-Benzyl-6-chloro-7H-purin
- Ausbeute: 0.14 g (6%)
- Schmelzpunkt: 150 °C
- IR (ATR): 1603, 1535, 1483, 1455, 1393, 1366, 1333, 1279, 1166, 1075, 966 794, 740,698 cm⁻¹
- ¹H-NMR (DMSO-*d*_{*6*}): δ (ppm) 5.76 (s, 2H, CH₂), 7.17-7.41 (m, 5H, Phenyl), 8.82 (s, 1H, C⁸-H), 9.01 (s, 1H, C²-H)
- ¹³C-NMR (DMSO-*d*_{*6*}): δ (ppm) 49.3 (CH₂), 121.9, 126.4 (2C, Phenyl), 127.8 (C^{4'}, Phenyl), 128.7 (2C, Phenyl), 136.6, 142.1, 151.3 (C⁸), 151.7 (C²), 161.6

### 6-Chloro-9-(4-fluoro-benzyl)-9H-purin (31) und 6-Chloro-7-(4-fluoro-benzyl)-7H-purin (32)

6-Chlor-9H-purin (1.55 g, 15 mmol) wird in 50 mL DMF gerührt, bis die Substanz vollständig gelöst ist. Man suspendiert K₂CO₃ (4.15 g, 30 mmol) und rührt 20 min. 4-Fluorbenzylchlorid (2.16 g, 15 mmol) wird langsam zugegeben und es wird 22 h bei 20 °C gerührt. Das Lösungsmittel wird abrotiert und der gelbe Rückstand säulenchromatographisch getrennt (SiO₂ 60, EtOAc/Hexan 670+330). Zuerst eluiert 6-Chloro-9-(4-fluoro-benzyl)-9H-purin und danach 6-Chloro-7-(4-fluoro-benzyl)-7H-purin.
- C₁₂H₈CIFN₄ (Mᵣ 262.68): 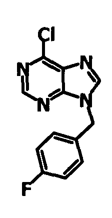 6-Chloro-9-(4-fluoro-benzyl)-9H-purin
- Ausbeute: 1.31 g (50%)
- Schmelzpunkt: 135 °C
- IR (ATR): 1593, 1557, 1511, 1445, 1385, 1344, 1244, 1220 (C-F), 1208, 1179, 1160, 1101, 938, 859, 755 cm⁻¹
- ¹H-NMR (DMSO-*d*_{*6*}): δ (ppm) 5.54 (s, 2H, CH₂), 7.15-7.23 (m, 2H, 4-F-Phenyl), 7.42-7.49 (m, 2H, 4-F-Phenyl), 8.80 (s, 1H, C⁸-H), 8.86 (s, 1H, C²-H)

- C₁₂H₈CIFN₄ (Mᵣ 262.68): 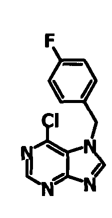 6-Chloro-7-(4-fluoro-benzyl)-7H-purin
- Ausbeute: 0.52 g (20%)
- Schmelzpunkt: 170 °C
- IR (ATR): 3059, 1599, 1543, 1511, 1450, 1391, 1367, 1330, 1292, 1216 (C-F), 1166, 1108, 973, 850, 827, 774, 752 cm⁻¹
- ¹H-NMR (DMSO-*d*_{*6*}): δ (ppm) 5.75 (s, 2H, CH₂), 7.15-7.33 (m, 4H, 4-F-Phenyl), 8.82 (s, 1H, C⁸-H), 9.0 (s, 1H, C²-H)

### 4-Chloro-5-nitro-6-phenyl-pyrimidin (33)

4,6-Dichlor-5-nitro-pyrimidin (2 g, 10.36 mmol), Phenylboronsäure (1.26 g, 10.36 mmol), Na₂CO₃ (3.29 g, 31 mmol), Pd(0)(PPh₃)₄ (200 mg, 0.17 mmol) werden in einem 50 mL Rundkolben vorgelegt. 8 mL Wasser und 10 mL Toluol werden hinzugefügt und das entstandene Zweiphasensystem 4.5 h unter Rückfluss gerührt. Das Gemisch wird mit 30 mL Wasser versetzt und dreimal mit je 60 mL Dichlormethan extrahiert. Die vereinigten Dichlormethanphasen werden über Na₂SO₄ getrocknet und einrotiert. Der braune, ölige Rückstand wird säulenchromatographisch gereinigt (SiO₂ 60, CH₂Cl₂).
- C₁₀H₆ClN₃O₂ (Mᵣ 235.63): 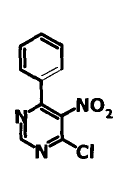
- Ausbeute: 1.54 g (63%)
- ¹H-NMR (DMSO-*d*_{*6*}): δ (ppm) 7.55-7.71 (m, 5H, Phenyl), 9.35 (s, 1H, C²-H)

### 4-Chloro-6-(4-fluoro-phenyl)-5-nitro-pyrimidin (34)

4,6-Dichlor-5-nitro-pyrimidin (2 g, 10.36 mmol), 4-Fluorphenylboronsäure (1.45 g, 10.36 mmol), Na₂CO₃ (3.29 g, 31 mmol), Pd(0)(PPh₃)₄ (200 mg, 0.17 mmol) werden in einem 50 mL Rundkolben vorgelegt. 10 mL Wasser und 25 mL Toluol werden hinzugefügt und das entstandene Zweiphasensystem wird 4 h unter Rückfluss gerührt. Das Gemisch wird mit 30 mL Wasser versetzt und dreimal mit je 60 mL Dichlormethan extrahiert. Die vereinigten Dichlormethanphasen werden über Na₂SO₄ getrocknet und einrotiert. Der braune, ölige Rückstand wird säulenchromatographisch gereinigt (SiO₂ 60, CH₂Cl₂).
- C₁₀H₅ClFN₃O₂ (Mᵣ 253.62): 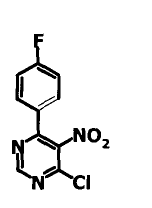
- Ausbeute: 1.58 g (60%)

### Methyl-(5-nitro-6-phenyl-pyrimidin-4-yl)-amin (35)

Eine Lösung von 4-Chloro-5-nitro-6-phenyl-pyrimidin (**33**) (1.54 g, 6.55 mmol) und 20 mL Methylaminlösung (40% in Wasser) in 25 mL Dioxan wird 1 h unter Rückfluss gerührt. Einrotieren liefert die Titelverbindung in quantitativer Ausbeute.
- C₁₁H₁₀N₄O₂ (Mᵣ 230.23): 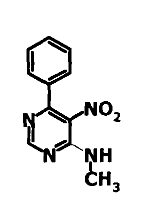
- Ausbeute: 1.5 g (100%)

### [6-(4-Fluoro-phenyl)-5-nitro-pyrimidin-4-yl]-methyl-amin (36)

Eine Lösung von 4-Chloro-6-(4-fluoro-phenyl)-5-nitro-pyrimidin (**34**) (1.58 g, 6.25 mmol) und 25 mL Methylaminlösung (40% in Wasser) in 25 mL Dioxan wird 1 h unter Rückfluss gerührt. Einrotieren liefert die Titelverbindung in quantitativer Ausbeute.
- C₁₁H₁₀N₄O₂ (Mᵣ 230.23): 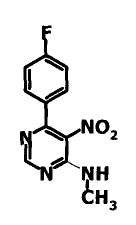
- Ausbeute: 1.55 g (100%)

### N'4'-Methyl-6-phenyl-pyrimidin-4,5-diamin (37)

Eine Lösung von FeSO₄ x 7 H₂O (18.7 g, 65 mmol) in 220 mL Wasser wird auf 75 °C erhitzt. Unter starkem Rühren wird Ba(OH)₂ x 8 H₂O (21.42 g, 68 mmol) portionsweise eingetragen und die entstehende Suspension auf 90 °C erhitzt und anschließend 20 min weitergerührt. Methyl-(5-nitro-6-phenyl-pyrimidin-4-yl)-amin (35) (1.5 g, 6.55 mmol) wird portionsweise eingetragen und die sich schwarz färbende Suspension bei 90 °C 20 min nachgerührt. Die Suspension wird heiß filtriert und mit 50mL kochendem Wasser nachgewaschen. Das Filtrat wird dreimal mit je 100 mL Dichlormethan ausgeschüttelt. Die vereinigten Dichlormethanphasen werden über Na₂SO₄ getrocknet und einrotiert.
- C₁₁H₁₂N₄ (Mᵣ 200.25): 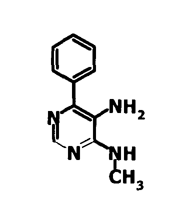
- Ausbeute: 600 mg (46%)
- ¹H-NMR (DMSO-*d*_{*6*}): δ (ppm) 2.91 (d, 3H, CH₃, ³J(H,H)=4.5 Hz, austauschbar), 4.52 (s, 2H, N-H₂), 6.73 (d, 1H, C⁴-N-H, ³J(H,H)=4.5 Hz, austauschbar), 7.37-7.67 (m, 5H, Phenyl), 8.03 (s, 1H, C²-H)

### 6-(4-Fluoro-phenyl)-N'4'-methyl-pyrimiclin-4,5-diamin (38)

Eine Lösung von FeSO₄ x 7 H₂O (18.7 g, 65 mmol) in 220 mL Wasser wird auf 75 °C erhitzt. Unter starkem Rühren wird Ba(OH)₂ x 8 H₂O (21.42 g, 68 mmol) portionsweise eingetragen und die entstehende Suspension auf 90 °C erhitzt und anschließend 20 min weitergerührt. [6-(4-Fluoro-phenyl)-5-nitro-pyrimidin-4-yl]-methyl-amin (36) (1.55 g, 6.25 mmol) wird portionsweise eingetragen und die sich schwarz färbende Suspension bei 90 °C 20 min nachgerührt. Die Suspension wird heiß filtriert und mit 50 mL kochendem Wasser nachgewaschen. Das Filtrat wird dreimal mit je 100 mL Dichlormethan ausgeschüttelt. Die vereinigten Dichlormethanphasen werden über Na₂SO₄ getrocknet und einrotiert.
- C₁₁H₁₁FN₄ (Mᵣ 218.24): 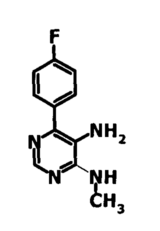
- Ausbeute: 1 g (73%)

### 9-Methyl-6-phenyl-9H-purin (39)

Eine Lösung von N'4'-Methyl-6-phenyl-pyrimidin-4,5-diamin (**37**) (600 mg, 3 mmol) in einem Gemisch aus 20 mL Orthoameisensäuretriethylester und 10 mL Ethanol wird mit einer Spatelspitze p-Toluolsulfonsäuremonohydrat versetzt und 5 h refluxiert. Das Lösungsmittel wird abrotiert und der grün-braune Rückstand säulenchromatographisch gereinigt (SiO₂ 60, CH₂Cl₂/EtOH 9+1).
- C₁₂H₁₀N₄ (Mᵣ 210.24): 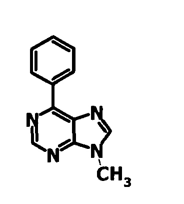
- Ausbeute: 300 mg (48%)
- Schmelzpunkt: 120 °C
- IR (ATR): 1579, 1566, 1509, 1322, 1235, 1201, 1051, 924, 862, 766, 737, 693, 663 cm⁻¹
- ¹H-NMR (DMSO-*d*_{*6*}): δ (ppm) 3.88 (s, 1H, N⁹-CH₃), 7.56-7.63 (m, 3H, Phenyl), 8.64 (s, 1H, C⁸-H), 8.83-8.88 (m, 2H, Phenyl), 9.00 (s, 1H, C²-H)
- ¹³C-NMR (DMSO-*d*_{*6*}): δ (ppm) 29.5 (N⁹-CH₃), 128.5 (2C, Phenyl), 128.9 (2C, Phenyl), 130.1, 130.9 (C⁴', Phenyl), 135.3, 147.0 (C⁸), 151.6 (C²), 152.2, 152.6

### 6-(4-Fluoro-phenyl)-9-methyl-9H-purin (40)

Eine Lösung von 6-(4-Fluoro-phenyl)-N'4'-methyl-pyrimidin-4,5-diamin (**38**) (500 mg, 2.29 mmol) in einem Gemisch aus 15 mL Orthoameisensäuretriethylester und 5 mL Ethanol wird mit einer Spatelspitze p-Toluolsulfonsäuremonohydrat versetzt und 5 h refluxiert. Das Lösungsmittel wird abrotiert und der grün-braune Rückstand säulenchromatographisch gereinigt (SiO₂ 60, EtOAc/Hexan 670+330).
- C₁₂H₉FN₄ (Mᵣ 228.23): 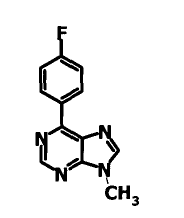
- Ausbeute: 190 mg (36%)
- Schmelzpunkt: 140 °C
- IR (ATR): 3058, 1601, 1574, 1515, 1452, 1393, 1322, 1218, 1169, 1049, 925, 852, 802, 735, 675, 663 cm⁻¹
- ¹H-NMR (DMSO-*d*_{*6*}): δ (ppm) 3.88 (s, 1H, N⁹-CH₃), 7.39-7.48 (m, 2H, 4-F-Phenyl), 8.64 (s, 1H, C⁸-H), 8.89-8.95 (m, 2H, 4-F-Phenyl), 8.98 (s, 1H, C²-H)

### 6-(4-Fluoro-phenyl)-9-methyl-8-phenyl-9H-purin (41)

Eine Lösung von 6-(4-Fluoro-phenyl)-N'4'-methyl-pyrimidin-4,5-diamin (**38**) (600 mg, 2.75 mmol) in einem Gemisch aus Orthobenzoesäuretriethylester (10 g, 44.6 mmol) und 5 mL Ethanol wird mit einer Spatelspitze p-Toluolsulfonsäuremonohydrat versetzt und 4 h refluxiert. Das Lösungsmittel wird abrotiert und der grün-braune Rückstand säulenchromatographisch gereinigt (SiO₂ 60, CH₂Cl₂/EtOH 9.5+0.5).
- C₁₈H₁₃FN₄ (Mᵣ 304.33): 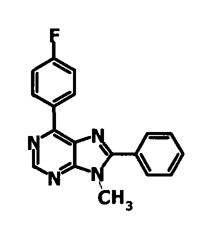
- Ausbeute: 310 mg (37%)
- Schmelzpunkt: 154°C
- IR (ATR): 1603, 1583, 1564, 1508, 1439, 1330, 1224, 1168, 983, 852, 804, 777, 705cm⁻¹
- ¹H-NMR (DMSO-*d*_{*6*}): δ (ppm) 3.93 (s, 1H, N⁹-CH₃), 7.38-7.67 (m, 5H, Phenyl), 7.98-8.02 (m, 2H, 4-F-Phenyl), 8.92-8.99 (m, 3H, 4-F-Phenyl, C²-H)

### 9-Benzyl-9H-purin-6-ylamin (42)

9-Benzyl-6-chloro-9H-purin (**29**) (400 mg, 1.64 mmol) wird in 40 mL ammoniakalischem Methanol (7 N Lösung) gelöst. Im geschlossenen Reaktionsgefäß wird bei 90 °C 7 h gerührt. Das Gefäß wird über Nacht im Kühlschrank stehen gelassen. Die Titelverbindung kristallisiert aus, wird abgesaugt und mit Diethylether gewaschen.
- C₁₂H₁₁N₅ (Mᵣ 225.25): 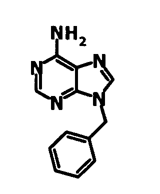
- Ausbeute: 240 mg (65%)
- Schmelzpunkt: 235 °C
- IR (ATR): 3087, 1642, 1594, 1570, 1484, 1412, 1324, 1299, 1243, 1067, 797, 776, 730, 692 cm⁻¹
- ¹H-NMR (DMSO-*d*_{*6*}): δ (ppm) 5.38 (s, 2H, CH₂), 7.27-7.36 (m, 7H, Phenyl, C⁶-NH₂ (austauschbar)), 8.17 (s, 1H, C²-H), 8.28 (s, 1H, C⁸-H)
- ¹³C-NMR (DMSO-*d*_{*6*}): δ (ppm) 46.0 (CH₂), 118.5, 127.4 (2C, Phenyl), 127.6 (C⁴', Phenyl), 128.5 (2C, Phenyl), 137.0, 140.7 (C⁸), 149.3, 152.5 (C²), 155.9

### 9-(4-Fluoro-benzyl)-9H-purin-6-ylamin (42a)

6-Chloro-9-(4-fluoro-benzyl)-9H-purin (31) (500 mg, 1.9 mmol) wird in 40 mL ammoniakalischem Methanol (7 N Lösung) gelöst. Im geschlossenen Reaktionsgefäß wird bei 90 °C 7 h gerührt. Das Gefäß wird über Nacht im Kühlschrank stehen gelassen. Die Titelverbindung kristallisiert aus, wird abgesaugt und mit Diethylether gewaschen.
- C₁₂H₁₀FN₅ (Mᵣ 243.25): 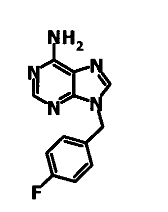
- Ausbeute: 350 mg (76%)
- Schmelzpunkt: 215 °C
- IR (ATR): 3095, 1648, 1597, 1511, 1486, 1424, 1355, 1325, 1300, 1247, 1224, 1154, 969, 824, 798, 778, 755, 720 cm⁻¹
- ¹H-NMR(DMSO-*d*_{*6*}): δ (ppm) 5.37 (s, 2H, CH₂), 7.13-7.43 (m, 6H, 4-F-Phenyl, C⁶-NH₂ (austauschbar)), 8.17 (s, 1H, C²-H), 8.28 (s, 1H, C⁸-H)

### 7-(4-Fluoro-benzyl)-7H-purin-6-ylamin (43) und 7-(4-Fluoro-benzyl)-6-methoxy-7H-purin (44)

6-Chloro-7-(4-fluoro-benzyl)-7H-purin (32) (200 mg, 0.76 mmol) wird in 40 mL ammoniakalischem Methanol (7 N Lösung) gelöst. Im geschlossenen Reaktionsgefäß wird bei 90 °C 7 h gerührt. Das Lösungsmittel wird abrotiert und der Rückstand säulenchromatographisch getrennt (Al₂O₃, CH₂Cl₂/EtOH 9+1). Zuerst eluiert 7-(4-Fluoro-benzyl)-6-methoxy-7H-purin und danach 7-(4-Fluoro-benzyl)-7Hpurin-6-ylamin.
- C₁₂H₁₀FN₅ (Mᵣ 243.25): 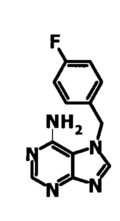 7-(4-Fluoro-benzyl)-7H-purin-6-ylamin
- Ausbeute: 20 mg (11 %)
- Schmelzpunkt: 220 °C
- IR (ATR): 3339, 3187, 1661, 1611, 1511, 1478, 1463, 1382 1216 (C-F), 1159, 1014, 817, 798, 708 cm⁻¹
- ¹H-NMR (DMSO-*d*_{*6*}): δ (ppm) 5.69 (s, 2H, CH₂), 7.05 (s, 2H, C⁶-NH₂, austauschbar), 7.13-7.22 (m, 4H, 4-F-Phenyl), 8.23 (s, 1H, C²-H), 8.47 (s, 1H, C⁸-H)
- ¹³C-NMR (DMSO-*d*_{*6*}): δ (ppm)

- C₁₃H₁₁FN₄O (Mᵣ 258.26): 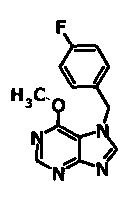 7-(4-Fluoro-benzyl)-6-methoxy-7H-purin
- Schmelzpunkt: 170 °C
- Ausbeute: 80 mg (41%)
- IR (ATR): 3068, 1613, 1555, 1507, 1481, 1395, 1354, 1324, 1216 (C-F), 1168, 1158, 1112, 965, 864, 769 cm⁻¹
- ¹H-NMR (DMSO-*d*_{*6*}): δ (ppm) 4.07 (s, 3H, O-CH₃), 5.55 (s, 2H, CH₂), 7.14-7.42 (m, 4H, 4-F-Phenyl), 8.54 (s, 1H, C⁸-H), 8.70 (s, 1H, C²-H)

### 9-Methyl-6-styryl-9H-purin (45)

6-Chloro-9-methyl-9H-purin (**12**) (144 mg, 0.85 mmol), trans-2-Phenylvinylboronsäure (189 mg, 1.28 mmol), K₂CO₃ (316 mg, 2.3 mmol), Pd(0)(PPh₃)₄ (28 mg, 0.02 mmol) werden in einem 50 mL Rundkolben vorgelegt. DME (Dimethoxyethan, 8 mL) wird hinzugefügt und es wird 3 h unter Rückfluss gerührt. Die ungelösten Anteile werden abfiltriert und das Filtrat einrotiert. Der braune, ölige Rückstand wird säulenchromatographisch gereinigt (SiO₂60, CH₂Cl₂/EtOH 9+1).
- C₁₄H₁₂N₄ (Mᵣ 236.28): 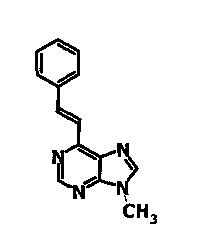
- Ausbeute: 32 mg (15%)
- Schmelzpunkt: 167 °C
- IR (ATR): 1636, 1575, 1442, 1323, 1225, 1190, 985, 804, 763, 724, 695 cm⁻¹
- ¹H-NMR (DMSO-*d*_{*6*}): δ (ppm) 3.87 (s, 1H, N⁹-CH₃), 7.41-7.51 (m, 3H, Phenyl), 7.65 (d, 1H, ³J(H,H)=16.2 Hz, trans), 7.77-7.82 (m, 2H, Phenyl), ), 8.42 (d, 1H, ³J(H,H)=16.2 Hz, trans), 8.59 (s, 1H, C⁸-H), 8.89 (s, 1H, C²-H)
- ¹³C-NMR (DMSO-*d*_{*6*}): δ (ppm) 29.4 (CH₃), 122.8, 127.6 (2C, Phenyl), 128.7, 129.4 (2C, Phenyl), 130.2, 135.5 138.9, 146.7 (C⁸), 151.6(C²), 151.9, 152.0

### 6-[2-(4-Fluoro-phenyl)-vinyl]-9-methyl-9H-purin (46)

6-Chloro-9-methyl-9H-purin (**12**) (432 mg, 2.57 mmol), trans-2-(4-Fluorphenyl)-vinylboronsäure (853 mg, 5.14 mmol), K₂CO₃ (966 mg, 7 mmol), Pd(0)(PPh₃)₄ (150 mg, 0.13 mmol) werden in einem 50 mL Rundkolben vorgelegt. DME (10 mL) wird hinzugefügt und es wird 24 h unter Rückfluss gerührt. Das Lösungsmittel wird abrotiert, der Rückstand in 50 mL Wasser aufgenommen und dreimal mit je 50 mL Dichlormethan extrahiert. Die vereinigten Dichlormethanphasen werden über Na₂SO₄ getrocknet und einrotiert. Der braune, ölige Rückstand wird säulenchromatographisch gereinigt (SiO₂ 60, CH₂Cl₂/EtOH 9.75+0.25).
- C₁₄H₁₁FN₄ (Mᵣ 254.27): 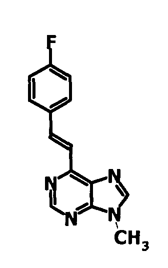
- ¹H-NMR (DMSO-*d*_{*6*}): δ (ppm) 3.87 (s, 1H, N⁹-CH₃), 7.63 (d, 1H, ³J(H,H)=16 Hz, trans), 7.72-7.82 (m, 4H, 4-F-Phenyl), 8.39 (d, 1H, ³J(H,H)=16 Hz, trans), 8,52 (s, 1H, C⁸-H), 8.87 (2, 1H, C²-H)

### 2-Cyano-4,4-diethoxy-butansäureethylester (47)

Zu einem Gemisch aus 2-Bromo-1,1-diethoxyethan (80 g, 0.4 mol) und Cyanessigsäureethylester (283 g, 2.5 mol) werden K₂CO₃ (56 g, 0.4 mol) und Nal (4 g, 26 mmol) gegeben. Die Suspension wird unter 6,5 h bei 145 °C gerührt. Der abgekühlte Ansatz wird in ein stark gerührtes Wasser-Ether-Gemisch (je 400 mL) gegossen. Die beiden Phasen werden im Scheidetrichter getrennt und die Etherphase wird zweimal mit 150 mL Wasser gewaschen. Die vereinigten Wasserphasen werden noch je zweimal mit 150 mL Ether extrahiert. Die Etherphasen werden vereinigt und über Na₂SO₄ getrocknet und einrotiert. Etwa 300 mL brauner, flüssiger Rückstand werden im Vakuum destilliert. Die Titelverbindung geht über bei 90-100 °C und 0,6 mbar.
- C₁₁H₁₉NO₄ (Mᵣ 229.28): 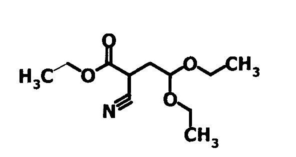
- Ausbeute: 19.5 g (21 %)
- ¹H-NMR (CDCl₃): δ (ppm) 1.15-1.24 (m, 6H, 2 * Acetal CH₃), 1.32 (t, 3H, ³J(H,H)=7.18 Hz, Ester CH₃), 2.18-2.31 (m, 2H, C³-H₂), 3.49-3.74 (m, 5H, 2 * Acetal CH₂, C²-H), 4.25 (q, 2H, ³J(H,H)=7.18 Hz, Ester CH₂), 4.69 (t, 1H, ³J(H,H)=5.56 Hz, C⁴-H)
- ¹³C-NMR (CDCl₃): δ (ppm) 13.5, 14.8, 14.9, 33.2, 33.3, 62.1, 62.2, 62.3, 99.6, 116.0, 165.6

### 6-Amino-5-(2,2-diethoxy-ethyl)-2-mercapto-pyrimidin-4-ol (48)

Natriumethanolat (5.8 g, 82 mmol) und Thioharnstoff (6.08 g, 80 mmol) werden unter Erwärmen in 100 mL Ethanol gelöst. 2-Cyano-4,4-diethoxy-butansäureethylester **(47)** (18.32 g, 80 mmol) wird zugegeben und 4 h unter Rückfluss gerührt. Das Lösungsmittel wird abrotiert und der Rückstand in 120 mL Wasser aufgenommen und mit 120 mL Ether extrahiert. Die Etherphase wird verworfen. Die dunkelbraune wässrige Phase wird unter Rühren mit Eisessig angesäuert. Die ausgefallenen weißen Kristalle werden abgesaugt, mit Wasser und Petrolether gewaschen und an der Luft getrocknet.
- C₁₀H₁₇N₃O₃S (Mr 259.33): 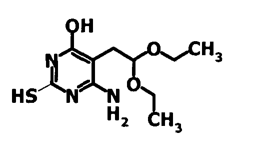
- Ausbeute: 11.3 g (55%)
- Schmelzpunkt: >315°C
- ¹H-NMR (DMSO-*d*_{*6*}): δ (ppm) 1.07 (t, 6H, 2 * Acetal CH₃), 2.44 (d, 2H, ³J(H,H)=5.6 Hz, CH₂), 3.34-3.63 (m, 4H, 2 * Acetal CH₂), 4.52 (t, 1H, ³J(H,H)=5.6 Hz, CH), 6.07 (s, 2H, NH₂), 11.45 (s, 1H, SH), 11.76 (s, 1H, OH)
- ¹³C-NMR (DMSO-*d*_{*6*}): δ (ppm) 16.6 (2C, 2 * CH₃), 28.2 (CH₂), 62.0 (2C, 2 * CH₂), 86.0, 102.0 (CH), 152.2, 162.1, 173.1

### 6-Amino-5-(2,2-diethoxy-ethyl)-pyrimidin-4-ol (49)

6-Amino-5-(2,2-diethoxy-ethyl)-2-mercapto-pyrimidin-4-ol (**48**) (11.1 g, 42 mmol), frisch hergestelltes Raney-Nickel (ca. 30 g ) und 60 mL Ammoniaklösung (25%-ig) werden in 500 mL Wasser 1 h unter Rückfluss gerührt. Es wird abgesaugt und das Filtrat einrotiert. Die zurückbleibenden weißen Kristalle werden im Luftstrom des Abzugs getrocknet.
- C₁₀H₁₇N₃O₃ (Mᵣ 227.27): 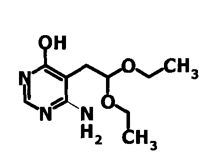
- Ausbeute: 8 g (84%)
- Schmelzpunkt: 188°C
- ¹H-NMR (DMSO-*d*_{*6*}): δ (ppm) 1.07 (t, 6H, 2 * Acetal CH₃), 2.53 (d, 2H, ³J(H,H)=5.6 Hz, CH₂), 3.32-3.68 (m, 4H, 2 * Acetal CH₂), 4.58 (t, 1H, ³J(H,H)=5.6 Hz, CH), 6.11 (s, 2H, NH₂), 7.71 (s, 1H, C²-H), 11.52 (s, 1H, OH)
- ¹³C-NMR (DMSO-*d*_{*6*}): δ (ppm) 15.2 (2C, 2 * CH₃), 28.6 (CH₂), 61.3 (2C, 2 * CH₂), 93.1, 101.8 (CH), 146.9 (C²), 161.3, 161.6

### 7H-Pyrrolo[2,3-d]pyrimidin-4-ol (50)

Eine Lösung von 6-Amino-5-(2,2-diethoxy-ethyl)-pyrimidin-4-ol (**49**) (7.8 g, 34.4 mmol) in 250 mL 0.2 N HCl wird 24 h bei Raumtemperatur gerührt. Die ausgefallene Titelverbindung wird abgesaugt, mit Wasser und Petrolether gewaschen und in der Trockenpistole unter Vakuum getrocknet.
- C₆H₅N₃O (Mᵣ 135.13): 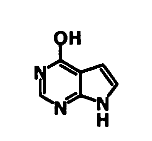
- Ausbeute: 4.04 g (87%)
- ¹H-NMR (DMSO-*d*_{*6*}): δ (ppm) 6.43 (1H, HC=CH), 7.02 (1H, HC=CH), 7.82 (s, 1H, C²-H), 11.78 (s, 1H, OH oder NH), 11.85 (s, 1H, OH oder NH)
- ¹³C-NMR (DMSO-*d*_{*6*}): δ (ppm) 102.3, 108.0, 120.6, 143.5, 148.4, 158.8

### 4-Chloro-7H-pyrrolo[2,3-d]pyrimidin (51)

Eine Lösung von 7H-Pyrrolo[2,3-d]pyrimidin-4-ol (**50**) (3.96 g, 29.3 mmol) in 50 mL POCl₃ wird 45 min unter Rückfluss gerührt. Das POCl₃ wird im Vakuum abdestilliert und der Rückstand auf Eis gegossen. Die wässrige Phase wird sechsmal mit je 100 mL Diethylether extrahiert. Die vereinigten Extrakte werden über NaSO₄ getrocknet und einrotiert. Die Titelverbindung fällt in Form hellgrüner Kristalle an.
- C₆H₄CIN₃ (Mᵣ 153.57): 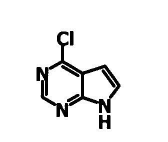
- Ausbeute: 3.5 g (78%)
- Schmelzpunkt: 168 °C
- ¹H-NMR (DMSO-*d*_{*6*}): δ (ppm) 6.62 (1H, HC=CH), 7.72 (1H, HC=CH), 8.61 (s, 1H, C²-H), 12.60 (s, 1H, NH)

### 4-Phenylsulfanyl-7H-pyrrolo[2,3-d]pyrimidin (52)

4-Chloro-7H-pyrrolo[2,3-d]pyrimidin (**51**) (765 mg, 5 mmol) wird in 30 mL Methanol unter Erwärmen gelöst. Eine Lösung von KOH (840 mg, 15 mmol) und Thiophenol (1.65 g, 15 mmol) in 15 mL Methanol wird dazugegeben und 30 min gerührt. Anschließend wird 1 h unter Rückfluss gerührt. 90 % des Lösungsmittels werden abrotiert. Mit Eisessig wird angesäuert, etwas Wasser zugegeben und mit je 50 mL Dichlormethan zweimal extrahiert. Die vereinigten Extrakte werden über NaSO₄ getrocknet und einrotiert. Der ölige Rückstand wird in etwas Diethylether aufgenommen und mit Hexan versetzt. Es setzt Kristallisation ein. Zur weiteren Kristallisation wird über Nacht im Kühlschrank stehen gelassen. Die Titelverbindung wird abgesaugt, mit Diethylether gewaschen und säulenchromatographisch gereinigt (SiO₂ 60, CH₂Cl₂/EtOH 9.75+0.25).
- C₁₂H₉N₃S (Mᵣ 227.29): 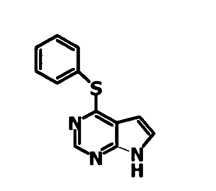
- Ausbeute: 624 mg (55%)
- Schmelzpunkt: 159 °C
- IR (ATR): 3190, 3117, 2988, 2835, 1588, 1554, 1438, 1350, 1269, 1222, 965, 842, 749, 688 cm⁻¹
- ¹H-NMR (DMSO-*d*_{*6*}): δ (ppm) 6.03 (1H, HC=CH), 7.45-7.69 (6H, Phenyl und HC=CH), 8.49 (s, 1H, C²-H), 12.27 (s, 1H, NH)
- ¹³C-NMR (DMSO-*d*_{*6*}): δ (ppm) 98.2, 114.8, 126.1, 128.0, 129.3 (2C, Phenyl), 129.4, 135.2 (2C, Phenyl), 149.8, 150.1, 159.4

### 4-(4-Fluoro-phenylsulfanyl)-7H-pyrrolo[2,3-d]pyrimidin (53)

4-Chloro-7H-pyrrolo[2,3-d]pyrimidin (**51**) (765 mg, 5 mmol) wird in 30 mL Methanol unter Erwärmen gelöst. Eine Lösung von KOH (840 mg, 15 mmol) und 4-Fluorthiophenol (1.92 g, 15 mmol) in 15 mL Methanol wird dazugegeben und 30 min gerührt. Anschließend wird 1 h unter Rückfluss gerührt. 75 % des Lösungsmittels werden abrotiert. Es setzt Kristallisation ein. Zur weiteren Kristallisation wird 2 h im Kühlschrank stehen gelassen. Die Titelverbindung wird abgesaugt, mit Diethylether gewaschen und aus Dichlormethan umkristallisiert.
- C₁₂H₈FN₃S (Mᵣ 245.28): 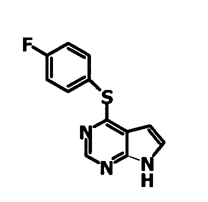
- Schmelzpunkt: 200 °C
- IR (ATR): 3189, 3108, 2970, 2831, 1586, 1553, 1490, 1354, 1268, 1212 (C-F), 1158, 968, 841, 823, 724 cm⁻¹
- ¹H-NMR (DMSO-*d*_{*6*}): δ (ppm) 6.12 (d, 1H, ³J(H,H)=3.5 Hz, HC=CH), 7.31-7.41 (2H, 4-F-Phenyl), 7.48 (d, 1H, ³J(H,H)=3.5 Hz, HC=CH), 7.66-7.75 (2H, 4-F-Phenyl), 8.47 (s, 1H, C²-H), 12.24 (s, 1H, NH)
- ¹³C-NMR (DMSO-*d*_{*6*}): δ (ppm) 98.1, 114.6, 116.2, 116.6, 123.4, 126.2, 137.7, 137.8, 149.7, 150.1, 159.2, 160.4, 165.3

### 8-Bromo-6-chloro-9-methyl-9H-purin (54)

Zu einer auf -78 °C abgekühlten Lösung von Diisopropylamin (1.70 g, 16.80 mmol) in THF abs. (15 mL) in einem ausgeheizten und mit Argon gespülten Dreihalskolben wird innerhalb von 15 min *n*-Butyllithium (15 %ige Lösung in *n*-Hexan, 7.17 g, 16.88 mmol) zugetropft. Nach beendeter Zugabe wird 1 h bei -78 °C gerührt. Innerhalb von 20 min wird tropfenweise eine Lösung von 6-Chloro-9-methyl-9H-purin (12) (2.02 g, 12 mmol) in THF abs. (20 mL) zugegeben und die Lösung nach beendeter Zugabe 1 h bei -78 °C gerührt. Innerhalb von 20 min wird eine Lösung von 1,2-Dibromtetrachlorethan (7.81 g, 24 mmol) in THF abs. (20 mL) zugetropft und die Lösung nach beendeter Zugabe 1 h bei -78 °C gerührt. Es wird mit gesättigter NH₄Cl-Lösung (10 mL) versetzt, auf Raumtemperatur erwärmt und das THF abrotiert. Zur zurückbleibenden wässrigen Phase werden weitere 50 mL Wasser gegeben und es wird mit je 50 mL Dichlormethan dreimal extrahiert. Die vereinigten Dichlormethanphasen werden über Na₂SO₄ getrocknet und einrotiert. Der Rückstand wird mit 100 mL Hexan kurz unter Rückfluss gerührt und heiß filtriert. Das Filtrat wird verworfen und der Rückstand säulenchromatographisch gereinigt (SiO₂ 60, CH₂Cl₂/EtOAc 6+4).
- C₆H₄BrCIN₄ (Mᵣ 247.48): 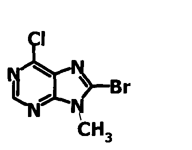
- Ausbeute: 1.96 g (66%)
- Schmelzpunkt: 187 °C
- ¹H-NMR (DMSO-*d*_{*6*}): δ (ppm) 3.7 (s, 3H, CH₃), 8.7 (s, 1H, C²-H)
- ¹³C-NMR (DMSO-*d*_{*6*}): δ (ppm) 30.9 (N⁹-CH₃), 130.9, 136.3, 147.1, 151.5 (C²), 152.9

### 6-Chloro-9-methyl-8-(4-methylsulfanyl-phenyl)-9H-purin (55)

8-Bromo-6-chloro-9-methyl-9H-purin (**54**) (500 mg, 2 mmol), 4-Methylthiophenylboronsäure (370 mg, 2.2 mmol), K₂CO₃ (345 mg, 2.5 mmol), Pd(0)(PPh₃)₄ (120 mg, 0.1 mmol) werden in einem 50 mL Rundkolben vorgelegt. Als Lösungsmittel wird Toluol (15 mL) hinzugefügt und es wird unter Argonatmosphäre und Lichtschutz 7.5 h bei 100 °C gerührt. Die ungelösten Anteile werden abfiltriert und das Filtrat einrotiert. Der braune, ölige Rückstand wird säulenchromatographisch gereinigt (SiO₂ 60, Hexan/EtOAc 1+1).
- C₁₃H₁₁ClN₄S (Mᵣ 290.78): 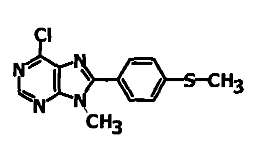
- Ausbeute: 350 mg (60%)
- Schmelzpunkt: 135 °C
- ¹H-NMR (DMSO-*d*_{*6*}): δ (ppm) 2.58 (s, 3H, S-CH₃), 3.95 (s, 3H, N⁹-CH₃), 7.45-7.49 (m, 2H, Phenyl), 7.45-7.49 (m, 2H, Phenyl), 8.78 (s, 1H, C²-H)
- ¹³C-NMR (DMSO-*d*_{*6*}): δ (ppm) 13.9 (S-CH₃), 31.2 (N⁹-CH₃), 124.3(C¹'), 125.3 (2C, C³'/C⁵'), 129.6 2C, C²'/C⁶'), 130.5 (C¹'), 142.6(C⁴'), 147.6, 151.0 (C²), 153.7, 153.6

### 9-Methyl-8-(4-methylsulfanyl-phenyl)-6-phenylsulfanyl-9H-purin (56)

Eine Lösung von 6-Chloro-9-methyl-8-(4-methylsulfanyl-phenyl)-9H-purin (**55**) (319 mg, 1.23 mmol), Thiophenol (288 mg, 2.6 mmol) und Triethylamin (298 mg, 2.95 mmol) in 10 mL n-Butanol wird 5 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, CH₂Cl₂/EtOH 9.75+0.25) und Trocknung unter Vakuum erhält man die Titelverbindung als weiße Substanz.
- C₁₉H₁₆N₄S₂ (Mᵣ 364.49): 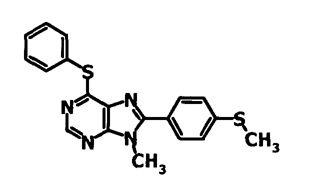
- Ausbeute: 223 mg (56%)
- Schmelzpunkt: 161 °C
- IR (ATR): 1597, 1570, 1475, 1430, 1399, 1339, 1245, 1157, 1098, 944, 829, 811, 752, 729, 691 cm⁻¹
- 1H-NMR (DMSO-d6): δ (ppm) 2.57 (s, 3H, S-CH3), 3.90 (s, 3H, N9-CH3), 7.44-7.52 (m, 5H, Phenyl), 7.63-7.68 (m, 2H, Phenyl), 7.87-7.91 (m, 2H, Phenyl), 8.58 (s, 1H, C2-H)
- ¹³C-NMR (DMSO-*d*_{*6*}): δ (ppm) 14.0 (S-CH3), 30.7 (N9-CH3), 124.8, 125.3 (2C, Phenyl), 126.9, 129.2 (2C, Phenyl), 129.3, 129.5 (2C, Phenyl), 129.8, 135.3 (2C, Phenyl), 142.0, 150.8, 151.1 (C2), 153.4, 157.2

### 6-(4-Fluoro-phenylsulfanyl)-9-methyl-8-(4-methylsulfanyl-phenyl)-9H-purin (57)

Eine Lösung von 6-Chloro-9-methyl-8-(4-methylsulfanyl-phenyl)-9H-purin (55) (320 mg, 1.1 mmol), 4-Fluorthiophenol (333 mg, 2.6 mmol) und Triethylamin (298 mg, 2.95 mmol) in 10 mL n-Butanol wird 4 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, CH₂Cl₂/EtOH 9.75+0.25) und Trocknung unter Vakuum erhält man die Titelverbindung als weiße Substanz.
- C₁₉H₁₅FN₄S₂ (Mᵣ 382.48): 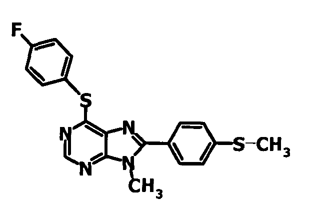
- Ausbeute: 280 mg (67%)
- Schmelzpunkt: 179°C
- IR (ATR): 1570, 1466, 1432, 1330, 1244, 1219(C-F), 1155, 1096, 947, 863, 830, 732, 698 cm⁻¹
- ¹H-NMR (DMSO-*d*_{*6*}): δ (ppm) 2.57 (s, 3H, S-CH₃), 3.90 (s, 3H, N⁹-CH₃), 7.31-7.48 (m, 4H, Phenyl), 7.67-7.74 (m, 2H, Phenyl), 7.86-7.91 (m, 2H, Phenyl), 8.58 (s, 1H, C²-H)
- ¹³C-NMR (DMSO-*d*_{*6*}): δ (ppm) 14.0 (S-CH3), 30.7 (N9-CH3), 116.4 (d, 2C, ²J(C,F)=22 Hz), 122.5 (d, 1C ⁴J(C,F)=2 Hz), 124.7, 125.3 (2C, Phenyl), 129.5 (2C, Phenyl), 129.7, 137.9 (d, 2C, ³J(C,F)=9 Hz), 142.0, 150.8, 151.1 (C2), 153.4, 157.2, 162.8 (d, 1C ¹J(C,F)=247 Hz)

### 6-Chloro-8-(4-methansulfinyl-phenyl)-9-methyl-9H-purin (58)

Zu einer Lösung von 6-Chloro-9-methyl-8-(4-methylsulfanyl-phenyl)-9H-purin **(55)** (660 mg, 2.27 mmol) in Eisessig (6 g) wird eine Lösung von H₂O₂ (30 %ige Lösung in Wasser, 1.9 g, 30 mmol) zugetropft. Das Reaktionsgemisch wird 3 h bei Raumtemperatur gerührt. Es wird Eis (ca. 30 g) zugegeben, mit 25 %igem Ammoniakwasser alkalisiert und zweimal mit je 50 mL Dichlormethan extrahiert. Die vereinigten Extrakte werden mit Wasser gewaschen, über Na₂SO₄ getrocknet und einrotiert.
- C₁₃H₁₁ClN₄OS (Mr 306.78): 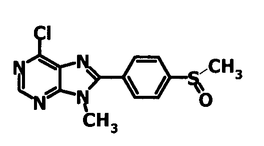
- Ausbeute: 620 mg (90%)
- ¹H-NMR (DMSO-*d*_{*6*}): δ (ppm) 2.87 (s, 3H, SO-CH₃), 3.98 (s, 3H, N⁹-CH₃), 7.93-7.97 (m, 2H, Phenyl), 8.17-8.21 (m, 2H, Phenyl), 8.53 (s, 1H, C²-H)
- ¹³C-NMR (DMSO-*d*_{*6*}): δ (ppm) 31.8 (N⁹-CH₃), 43.6 (SO-CH₃), 124.7 (2C, C³'/C⁵'), 130.8 2C, C²'/C⁶'), 131.0 (C¹'), 131.1 (C⁴'), 148.9, 149.9, 152.0 (C²), 154.3, 155.7

### 8-(4-Methansulfinyl-phenyl)-9-methyl-6-phenylsulfanyl-9H-purin (59).

Eine Lösung von 6-Chloro-8-(4-methansulfinyl-phenyl)-9-methyl-9H-purin (**58**) (300 mg, 0.98 mmol), Thiophenol (288 mg, 2.6 mmol) und Triethylamin (298 mg, 2.95 mmol) in 10 mL n-Butanol wird 4 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, CH₂Cl₂/EtOH 9.5+0.5) und Trocknung unter Vakuum erhält man die Titelverbindung als gelblich weiße Substanz.
- C₁₉H₁₆N₄OS₂ (Mᵣ 380.49): 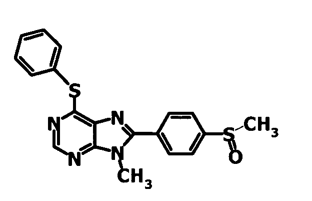
- Ausbeute: 180 mg (50%)
- Schmelzpunkt: 196 °C
- IR (ATR): 1570, 1556, 1471, 1438, 1398, 1335, 1250, 1209, 1156, 1087, 1043 (S=O), 1011, 947, 867, 836, 792, 750, 732, 702, 687 cm⁻¹
- ¹H-NMR (DMSO-*d*_{*6*}): δ (ppm) 2.87 (s, 3H, SO-CH₃), 3.95 (s, 3H, N⁹-CH₃), 7.50-7.70 (m, 5H, Phenyl), 7.92-7.96 (m, 2H, Phenyl), 8.15-8.19 (m, 2H, Phenyl), 8.64 (s, 1H, C²-H)
- ¹³C-NMR (DMSO-*d*_{*6*}): δ (ppm) 31.4 (N9-CH3), 46.6 (SO-CH3), 124.6 (2C, Phenyl), 127.4, 129.9 (2C, Phenyl), 130.0 (C⁶-Phenyl, C⁴'), 130.4, 130.6 2C, Phenyl), 131.5, 136.4 (2C, Phenyl), 149.5, 151.4, 152.1 (C2), 153.4, 158.6

### 6-(4-Fluoro-phenylsulfanyl)-8-(4-methan-sulfinyl-phenyl)-9-methyl-9H-purin (60).

Eine Lösung von 6-Chloro-8-(4-methansulfinyl-phenyl)-9-methyl-9H-purin (58) (300 mg, 0.98 mmol), 4-Fluorthiophenol (333 mg, 2.6 mmol) und Triethylamin (298 mg, 2.95 mmol) in 10 mL n-Butanol wird 4 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, CH₂Cl₂/EtOH 9.5+0.5) und Trocknung unter Vakuum erhält man die Titelverbindung als gelblich weiße Substanz.
- C₁₉H₁₅FN₄OS₂ (Mᵣ 398.48): 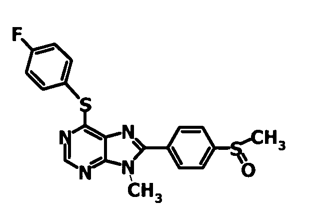
- Schmelzpunkt: 213 °C
- IR (ATR): 1574, 1474, 1397, 1331, 1248, 1217, 1154, 1087, 1048 (S=O), 1011, 947, 834, 811, 734 cm⁻¹
- ¹H-NMR (DMSO-*d*_{*6*}): δ (ppm) 2.85 (s, 3H, SO-CH₃), 3.93 (s, 3H, N⁹-CH₃), 7.32-7.17 (m, 8H, Phenyl), 8.62 (s, 1H, C²-H)
- ¹³C-NMR (DMSO-*d*_{*6*}): δ (ppm) 30.7 (N9-CH3), 43.0 (SO-CH3), 116.2, 116.6, 122.2 (2C, Phenyl), 129.7, 129.9 (2C, Phenyl), 130.8, 137.8, 138.0, 148.8, 150.7, 151.5, 152.7, 157.8, 160.4, 165.3

### 6-Chloro-8-(4-methansulfonyl-phenyl)-9-methyl-9H-purin (61)

Zu einer Lösung von 6-Chloro-9-methyl-8-(4-methylsulfanyl-phenyl)-9H-purin (**55**) (1,1 g, 3.8 mmol) in Dichlormethan (20 mL) wird 3-Chlorperbenzoesäure (2 g, 8.14 mmol) gegeben. Das Reaktionsgemisch wird 4 h unter Rückfluss gerührt. Es wird zweimal mit je 30 mL gesättigter NaHCO₃-Lösung und zweimal mit je 30 mL Wasser gewaschen. Die Dichlormethanphase wird über Na₂SO₄ getrocknet und einrotiert.
- C₁₃H₁₁ClN₄O₂S (Mᵣ 322.78): 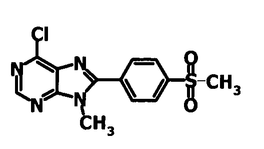
- Ausbeute: 1.16 g (95%)
- ¹H-NMR (DMSO-*d*_{*6*}): δ (ppm) 3.34 (s, 3H, SO-CH₃), 3.97 (s, 3H, N⁹-CH₃), 8.21 (q, 4H, Phenyl), 8.85 (s, 1H, C²-H)

### 8-(4-Methansulfonyl-phenyl)-9-methyl-6-phenylsulfanyl-9H-purin (62).

Eine Lösung von 6-Chloro-8-(4-methansulfonyl-phenyl)-9-methyl-9H-purin (**61**) (500 mg, 1.55 mmol), Thiophenol (444 mg, 4 mmol) und Triethylamin (462 mg, 4.6 mmol) in 25 mL n-Butanol wird 4 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, CH₂Cl₂/EtOH 9.75+0.25) und Trocknung unter Vakuum erhält man die Titelverbindung als gelblich weiße Substanz.
- C₁₉H₁₆N₄O₂S₂ (Mᵣ 396.49): 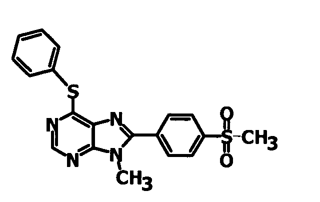
- Ausbeute: 260 mg (42%)
- Schmelzpunkt: 241 °C
- IR (ATR): 1572, 1555, 1397, 1339, 1302, 1246, 1147 (S=O₂), 947, 783, 763, 724, 692 cm⁻¹
- ¹H-NMR (DMSO-*d*_{*6*}): δ (ppm) 3.34 (s, 3H, SO-CH₃), 3.94 (s, 3H, N⁹-CH₃), 7.51-7.69 (m, 5H, Phenyl), 8.20 (q, 4H, Phenyl an C⁸), 8.64 (s, 1H, C²-H)
- ¹³C-NMR (DMSO-*d*_{*6*}): δ (ppm) 31.7 (N9-CH3), 43.2 (SO₂-CH3), 126.6, 127.3 (2C, Phenyl), 129.3 (2C, Phenyl), 129.4 (C⁶-Phenyl, C⁴'), 129.8, 130.1 (2C, Phenyl), 133.5, 135.4 (2C, Phenyl), 142.3, 150.7, 151.7, 152.1 (C2), 158.3

### 6-(4-Fluoro-phenylsulfanyl)-8-(4-methan-sulfonyl-phenyl)-9-methyl-9H-purin (63).

Eine Lösung von 6-Chloro-8-(4-methansulfonyl-phenyl)-9-methyl-9H-purin (**61**) (500 mg, 1.55 mmol), 4-Fluorthiophenol (512 mg, 4 mmol) und Triethylamin (462 mg, 4.6 mmol) in 25 mL n-Butanol wird 4 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, CH₂Cl₂/EtOH 9.75+0.25) und Trocknung unter Vakuum erhält man die Titelverbindung als gelblich weiße Substanz.
- C₁₉H₁₅FN₄O₂S₂ (Mᵣ 414.48): 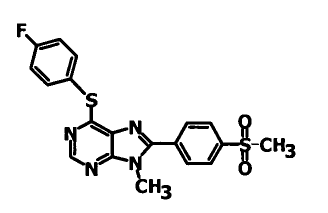
- Ausbeute: 150 g (23%)
- Schmelzpunkt: 250 °C
- IR (ATR): 1572, 1559, 1473, 1399, 1212 (C-F), 1248, 1149 (S=O₂), 1090, 946, 935, 782, 737 cm⁻¹
- ¹H-NMR (CDCl₃): δ (ppm) 3.13 (s, 3H, SO-CH₃), 3.99 (s, 3H, N⁹-CH₃), 7.14-7.26 (m, 2H, 4-F-Phenyl), 7.62-7.69 (m, 2H, 4-F-Phenyl), 8.12 (q, 4H, Phenyl an C⁸), 8.66 (s, 1H, C²-H)
- ¹³C-NMR (CDCl₃): δ (ppm) 31.0 (N9-CH3), 44.4 (SO-CH3), 116.4, 116.8, 122.2, 128.0 (2C, Phenyl an C⁸), 130.3 (2C, Phenyl an C⁸), 130.6, 134.3, 137.7, 137.9, 142.3, 151.0, 151.6, 152.4 (C²), 160.5, 161.2, 166.2

### 6-Chloro-9-(tetrahydro-pyran-2-yl)-9H-purin (64)

Eine Lösung von 6-Chloro-9H-purin (3 g, 19.5 mmol) und p-Toluolsulfonsäuremonohydrat (90 mg, 0.47 mmol) in Ethylacetat (45 mL) wird unter Rühren auf 60 °C erwärmt. Innerhalb von 30 min wird tropfenweise 3,4-Dihydro-2H-pyran (3 g, 36 mmol) zugegeben und 30 min bei 60 °C, dann 1 h bei Raumtemperatur gerührt. Es wird 25 %iges Ammoniakwasser (ca. 2 mL) zugegeben, 5 min gerührt, dreimal mit Wasser (je 50 mL) gewaschen, über Na₂SO₄ getrocknet und einrotiert. Der Rückstand wird sechsmal mit siedendem Petrolether (je 15 mL) extrahiert. Die vereinigten Extrakte werden zur Kristallisation auf Eis gestellt. Die gelblich weißen Kristalle werden abgesaugt und mit Petrolether gewaschen.
- C₁₀H₁₁ClN₄O (Mr 238.68): 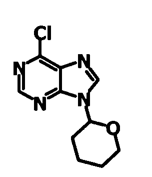
- Ausbeute: 3.64 g (78%)
- Schmelzpunkt: 63 °C
- ¹H-NMR (CDCl₃): δ (ppm) 1.69-1.80 (m, 3H), 2.07-2.16 (m, 3H), 3.73-3.86 (m, 1H), 4.16-4.24 (m, 1H), 5.77-5.83 (m, 1H), 8.35 (s, 1H, C⁸-H ), 8.76 (s, 1H, C²-H ),
- ¹³C-NMR (CDCl₃): δ (ppm) 22.6, 24.4, 31.8, 68.9, 82.5 (C^{2'}), 131.7, 143.0 (C⁸), 151.0, 151.1, 152.0 (C²)

### 8-Bromo-6-chloro-9-(tetrahydro-pyran-2-yl)-9H-purin (65)

Zu einer auf -78 °C abgekühlten Lösung von Diisopropylamin (1.84 g, 18.20 mmol) in THF abs. (20 mL) in einem ausgeheizten und mit Argon gespülten Dreihalskolben wird innerhalb von 15 min *n*-Butyllithium (15 %ige Lösung in *n*-Hexan, 7.8 g, 18.29 mmol) zugetropft. Nach beendeter Zugabe wird 1 h bei -78 °C gerührt. Innerhalb von 20 min wird tropfenweise eine Lösung von 6-Chloro-9-(tetrahydro-pyran-2-yl)-9Hpurin (**64**) (3.1 g, 13 mmol) in THF abs. (20 mL) zugegeben und die Lösung nach beendeter Zugabe 1 h bei -78 °C gerührt. Innerhalb von 20 min wird eine Lösung von 1,2-Dibromtetrachlorethan (8.47 g, 26 mmol) in THF abs. (20 mL) zugetropft und die Lösung nach beendeter Zugabe 1 h bei -78 °C gerührt. Es wird mit gesättigter NH₄Cl-Lösung (10 mL) versetzt, auf Raumtemperatur erwärmt und das THF abrotiert. Zur zurückbleibenden wässrigen Phase werden weitere 50 mL Wasser gegeben und es wird mit je 50 mL Dichlormethan dreimal extrahiert. Die vereinigten Dichlormethanphasen werden über Na₂SO₄ getrocknet und einrotiert. Der Rückstand wird mit 100 mL Hexan kurz unter Rückfluss gerührt und heiß filtriert. Das Filtrat wird verworfen und der Rückstand säulenchromatographisch gereinigt (SiO₂ 60, CH₂Cl₂/EtOAc 6+4).
- C₁₀H₁₀BrClN₄O (Mr 317.57): 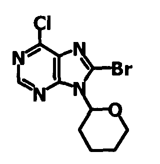
- Ausbeute: 2.73 g (55%)
- Schmelzpunkt: 110°C
- ¹H-NMR (CDCl₃): δ (ppm) 1.51-2.13 (m, 6H), 3.68-3.79 (m, 1H), 4.18-4.24 (m, 1H), 5.71-5.78 (m, 1H), 8.73 (s, 1H, C²-H)
- ¹³C-NMR (CDCl₃): δ (ppm) 23.2, 24.6, 28.7, 69.3, 85.6 (C²'), 132.0, 133.4, 149.7, 151.8, 152.6 (C²)

### 6-Chloro-8-(4-methylsulfanyl-phenyl)-9-(tetrahydro-pyran-2-yl)-9H-purin (66)

8-Bromo-6-chloro-9-(tetrahydro-pyran-2-yl)-9H-purin (65) (950 mg, 3 mmol), 4-Methylthiophenylboronsäure (555 mg, 3.3 mmol), K₂CO₃ (520 mg, 3.75mmol), Pd(0)(PPh₃)₄ (180 mg, 0.16 mmol) werden in einem 50 mL Rundkolben vorgelegt. Als Lösungsmittel wird Toluol (20 mL) hinzugefügt und es wird unter Argonatmosphäre und Lichtschutz 5 h bei 100 °C gerührt. Die ungelösten Anteile werden abfiltriert und das Filtrat einrotiert. Der braune, ölige Rückstand wird säulenchromatographisch gereinigt (SiO₂ 60, Hexan/EtOAc 1+1).
- C₁₇H₁₇ClN₄OS (Mᵣ 360.87): 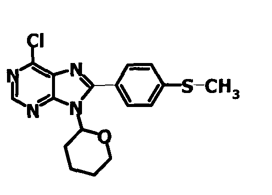
- Ausbeute: 440 mg (41 %)
- ¹H-NMR (CDCl₃): δ (ppm) 1.55-1.80 (m, 6H), 3.68-3.74 (m, 1H), 4.22-4.24 (m, 1H), 5.54-5.60 (m, 1H), 7.36-7.42 (m, 2H, Phenyl), 7.78-7.85 (m, 2H, Phenyl), 8.75 (s, 1H, C²-H)
- ¹³C-NMR (CDCl₃): δ (ppm) 14.9 (S-CH₃), 23.2, 24.5, 28.3, 68.8, 84.4, 125.0, 125.5 (2C, Phenyl), 130.0 (2C, Phenyl), 143.2, 150.1, 151.2 (C²), 153.2, 156.1

### 8-(4-Methylsulfanyl-phenyl)-6-phenylsulfanyl-9H-purin (67)

Eine Lösung von 6-Chloro-8-(4-methylsulfanyl-phenyl)-9-(tetrahydro-pyran-2-yl)-9Hpurin (**66**) (410 mg, 1.13 mmol), Thiophenol (333 mg, 3 mmol) und Triethylamin, (348 mg, 3.45 mmol) in 10 mL n-Butanol wird 4 h unter Rückfluss gerührt. Das Lösungsmittel wird abrotiert. Der Rückstand wird in einen Büchnertrichter überführt, mit Hexan gewaschen und in einem Gemisch aus Ethanol (25mL) und 1 N HCl (10 mL) 4 h gerührt. Die überschüssige Säure wird durch Zugabe von NaHCO₃ (ca. 1 g) neutralisiert und das Lösungsmittel wird abrotiert. Nach säulenchromatographischer Reinigung (SiO₂ 60, CH₂Cl₂/EtOH 9.75+0.25) und Trocknung unter Vakuum erhält man die Titelverbindung als gelblich weiße Substanz.
- C₁₈H₁₄N₄S₂ (Mᵣ 350.47): 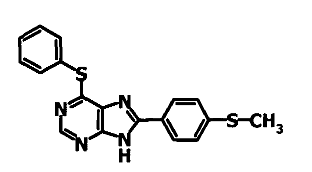
- ¹³C-NMR (DMSO-*d*_{*6*}): δ (ppm) 14.7 (S-CH3), 125.4, 126.2 (2C, Phenyl), 127.7 (2C, Phenyl), 127.9, 129.9 (2C, Phenyl), 130.0, 131.5, 136.0 (2C, Phenyl), 143.1, 152.0 (C2), 152.2, 152.6, 157.3

### Benzyl-tributyl-phosphoniumbromid (68)

Eine Lösung von Tributylphosphin (5.05 g, 25 mmol) und Benzylbromid (4.28 g, 25 mmol) in Toluol (50 mL) wird 4 h unter Rückfluss gerührt. Der Ansatz wird über Nacht abkühlen gelassen. Die ausgefallene Titelverbindung wird abgesaugt und mit Hexan gewaschen.
- C₁₉H₃₄BrP (Mᵣ 373.36): 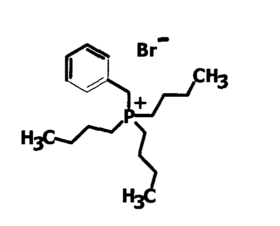
- Ausbeute: 8.4 g (90%)
- ¹H-NMR (CDCl₃): δ (ppm) 0.88-0.95 (m, 9H), 1.41-1.51 (m, 12H), 2.35-2.49 (m, 6H), 4.27 (d, 2H, CH₂), 7.30-7.50 (m, 5H, Phenyl)

### Tributyl-(4-fluoro-benzyl)-phosphonium bromid (69)

Eine Lösung von Tributylphosphin (5.45 g, 27 mmol) und 4-Fluorbenzylbromid (5.1 g, 25 mmol) in Toluol (50 mL) wird 4 h unter Rückfluss gerührt. Der Ansatz wird über Nacht abkühlen gelassen. Die ausgefallene Titelverbindung wird abgesaugt und mit Hexan gewaschen.
- C₁₉H₃₃BrFP (Mᵣ 391.35): 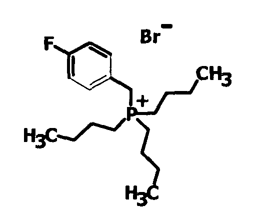
- Ausbeute: 9.6 g (91 %)
- ¹H-NMR (DMSO-*d*_{*6*}): δ (ppm) 0.86-0.93 (m, 9H), 1.37-1.49 (m, 12H), 2.14-2.29 (m, 6H), 3.95 (d, 2H, CH₂), 7.24-7.50 (m, 4H, 4-F-Phenyl)

### 6-Benzyl-9H-purin (70)

Benzyl-tributyl-phosphoniumbromid (68) (3.42 g, 9.2 mmol) wird in einem ausgeheizten und mit Argon gespülten Dreihalskolben in DME (50 mL) suspendiert. Die gerührte Suspension wird auf -35 °C abgekühlt. Innerhalb von 10 min wird n-Butyllithium (15%ige Lösung in n-Hexan, 4g, 9.25 mmol) zugetropft. Nach beendeter Zugabe wird 1 h bei -35 °C gerührt. Innerhalb von 10 min wird tropfenweise eine Lösung von 6-Chloro-9-(tetrahydro-pyran-2-yl)-9H-purin (64) (1 g, 4.2 mmol) in DME (10 mL) zugegeben. Nach beendeter Zugabe wird der Kryostat ausgeschaltet und die Lösung unter weiterem Rühren über ca. 1 h auf Raumtemperatur erwärmen gelassen. Eine Lösung von NaHCO₃ (0.44 g, 4.2 mmol) in Wasser (10 mL) wird zugegeben, über Nacht gerührt und morgens 2 h unter Rückfluss gerührt. Das Lösungsmittel wird abrotiert, der Rückstand wird in Wasser (50 mL) aufgenommen und dreimal mit Diethylether (je 50 mL) extrahiert. Die vereinigten Extrakte werden über Na₂SO₄ getrocknet und einrotiert. Der gelbe, zähe Rückstand wird in in einem Gemisch aus Ethanol (25mL) und 1 N HCl (10 mL) aufgenommen und 4 h gerührt. Die überschüssige Säure wird durch Zugabe von NaHCO₃ (ca. 1 g) neutralisiert und das Lösungsmittel wird abrotiert. Um die anorganischen Bestandteile abzutrennen, wird der Rückstand in Dichlormethan aufgenommen, filtriert und einrotiert. Es wird säulenchromatographisch gereinigt (SiO₂ 60, CH₂Cl₂/EtOH 9.5+0.5).
- C₁₂H₁₀N₄ (Mᵣ 210.24): 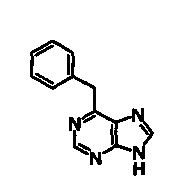
- Schmelzpunkt: 150°C
- IR (ATR): 3060, 2966, 2809,1589, 1424, 1403, 1230, 926, 894, 836, 763, 707 cm⁻¹
- ¹H-NMR (CDCl₃): δ (ppm) 4.59 (s, 2H, CH₂), 7.19-7.45 (m, 5H, Phenyl), 8.24 (s, 1H, C⁸-H), 8.98 (s, 1H, C²), N⁹-H nicht sichtbar
- ¹³C-NMR (CDCl₃): δ (ppm) 39.6 (CH₂), 126.8 (C⁴', Phenyl), 128.7 (2C, Phenyl), 129.3 (2C, Phenyl), 131.6, 137.4, 142.8 (C⁸), 152.0 (C²), 152.4, 160.6

### 6-(4-Fluoro-benzyl)-9H-purin (71)

Tributyl-(4-fluoro-benzyl)-phosphonium bromid (**69**) (3.6 g, 9.2 mmol) wird in einem ausgeheizten und mit Argon gespülten DHK in DME (50 mL) suspendiert. Die gerührte Suspension wird auf -35 °C abgekühlt. Innerhalb von 10 min wird *n*-BuLi (15 %ige Lösung in *n*-Hexan, 4 g, 9.25 mmol) zugetropft. Nach beendeter Zugabe wird 1 h bei -35 °C gerührt. Innerhalb von 10 min wird tropfenweise eine Lösung von 6-Chloro-9-(tetrahydro-pyran-2-yl)-9H-purin (64) (1 g, 4.2 mmol) in DME (10 mL) zugegeben. Nach beendeter Zugabe wird der Kryostat ausgeschaltet und die Lösung unter weiterem Rühren über ca. 1 h auf Raumtemperatur erwärmen gelassen. Eine Lösung von NaHCO₃ (0.44 g, 4.2 mmol) in Wasser (10 mL) wird zugegeben, über Nacht gerührt und morgens 2 h unter Rückfluss gerührt. Das Lösungsmittel wird abrotiert, der Rückstand wird in Wasser (50 mL) aufgenommen und dreimal mit Diethylether (je 50 mL) extrahiert. Die vereinigten Extrakte werden über Na₂SO₄ getrocknet und einrotiert. Der gelbe, zähe Rückstand wird in in einem Gemisch aus Ethanol (25mL) und 1 N HCl (10 mL) aufgenommen und 4 h gerührt. Die überschüssige Säure wird durch Zugabe von NaHCO₃ (ca. 1 g) neutralisiert und das Lösungsmittel wird abrotiert. Um die anorganischen Bestandteile abzutrennen, wird der Rückstand in Dichlormethan aufgenommen, filtriert und einrotiert. Es wird säulenchromatographisch gereinigt (SiO₂ 60, CH₂Cl₂/EtOH 9.5+0.5).
- C₁₂H₁₀N₄ (Mᵣ 210.24): 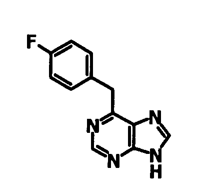
- Ausbeute: 228 mg (24 %)
- Schmelzpunkt: 176 °C
- IR (ATR): 3053, 2964, 2809, 1592, 1509, 1427, 1402, 1328, 1228 (C-F), 1158, 926, 895, 856, 814, 762, 730, 696 cm⁻¹
- ¹H-NMR (CDCl₃): δ (ppm) 4.55 (s, 2H, CH₂), 6.92-7.01 (m, 2H, 4-F-Phenyl), 7.39-7.46 (m, 2H, 4-F-Phenyl), 8.29 (s, 1H, C⁸-H), 8.96 (s, 1H, C²), 12.75 (s, 1H, N⁹-H)
- ¹³C-NMR (CDCl₃): δ (ppm) 38.7 (CH₂), 115.2, 115.6, 130.7, 130.9, 132.2, 133.2, 142.2 (C⁸), 151.7, 152.1 (C²), 159.3, 161.0, 164.2

### 6-Styryl-9H-purin (72)

Methyl-triphenyl-phosphonium bromid (3.28 g, 9.2 mmol) wird in einem ausgeheizten und mit Argon gespülten DHK in DME (50 mL) suspendiert. Die gerührte Suspension wird auf -35 °C abgekühlt. Innerhalb von 10 min wird n-BuLi (15 %ige Lösung in n-Hexan, 4 g, 9.25 mmol) zugetropft. Nach beendeter Zugabe wird 1 h bei -35 °C gerührt. Innerhalb von 10 min wird tropfenweise eine Lösung von 6-Chloro-9-(tetrahydro-pyran-2-yl)-9H-purin (**64**) (1 g, 4.2 mmol) in DME (10 mL) zugegeben. Nach beendeter Zugabe wird der Kryostat ausgeschaltet und die Lösung unter weiterem Rühren über ca. 1 h auf Raumtemperatur erwärmen gelassen und 4 h gerührt. Eine Lösung von Benzaldehyd (1.78 g, 16.8 mmol) in DME (5 mL) wird zugegeben und 24 h gerührt. Es wird abfiltriert und das Filtrat einrotiert. Der gelbe, zähe Rückstand wird in in einem Gemisch aus Ethanol (25 mL) und 1 N HCl (10 mL) aufgenommen und 4 h bei Raumtemperatur gerührt. Die überschüssige Säure wird durch Zugabe von NaHCO₃ (ca. 1 g) neutralisiert und das Lösungsmittel wird abrotiert. Um die anorganischen Bestandteile abzutrennen, wird der Rückstand in Dichlormethan aufgenommen, filtriert und einrotiert. Es wird säulenchromatographisch gereinigt (SiO₂ 60, CH₂Cl₂/EtOH 9.5+0.5).
- C₁₃H₁₀N₄ (Mᵣ 222.25): 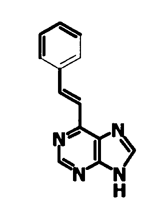
- Ausbeute: 185 mg (20%)
- Schmelzpunkt: 207 °C
- IR (ATR): 3056, 2982, 2823, 1637, 1602, 1587, 1448, 1323, 1255, 1123, 1044, 987, 973, 923, 836, 802, 749, 689 cm⁻¹
- ¹H-NMR (DMSO-*d*_{*6*}): δ (ppm) 7.37-7.52 (m, 3H), 7.65-7.81 (m, 3H), 8.63 (s, 1H, C⁸-H), 8.86 (s, 1H, C²), 13.56 (s, 1H, N⁹-H)

### 6-[2-(4-Fluoro-phenyl)-vinyl]-9H-purin (73)

Methyl-triphenyl-phosphonium bromid (6.56 g, 18.4 mmol) wird in einem ausgeheizten und mit Argon gespülten DHK in DME (50 mL) suspendiert. Die gerührte Suspension wird auf -35 °C abgekühlt. Innerhalb von 15 min wird n-BuLi (15 %ige Lösung in n-Hexan, 8 g, 18.5 mmol) zugetropft. Nach beendeter Zugabe wird 1 h bei -35 °C gerührt. Innerhalb von 15 min wird tropfenweise eine Lösung von 6-Chloro-9-(tetrahydro-pyran-2-yl)-9H-purin (**64**) (2 g, 8.4 mmol) in DME (20 mL) zugegeben. Nach beendeter Zugabe wird der Kryostat ausgeschaltet und die Lösung unter weiterem Rühren über ca. 1 h auf Raumtemperatur erwärmen gelassen und 4 h gerührt. Eine Lösung von 4-Fluorbenzaldehyd (4.17 g, 33.6 mmol) in DME (5 mL) wird zugegeben und 24 h gerührt. Es wird abfiltriert und das Filtrat einrotiert. Der gelbe, zähe Rückstand wird in in einem Gemisch aus Ethanol (50 mL) und 1 N HCl (20 mL) aufgenommen und 4 h bei Raumtemperatur gerührt. Die überschüssige Säure wird durch Zugabe von NaHCO₃ (ca. 2 g) neutralisiert und das Lösungsmittel wird abrotiert. Um die anorganischen Bestandteile abzutrennen, wird der Rückstand in Ethanol aufgenommen, filtriert und einrotiert. Es wird säulenchromatographisch gereinigt (SiO₂ 60, CH₂Cl₂/EtOH 9.5+0.5).
- C₁₃H₁₀N₄ (Mᵣ 222.25): 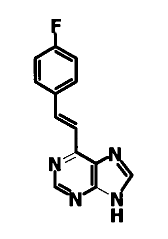
- ¹H-NMR (DMSO-*d*_{*6*}): δ (ppm) 7.25-7.79 (m, 6H), (s, 1H, C⁸-H), 8.76 (s, 1H, C²), 13,61 (1H, N⁹-H)
- ¹³C-NMR (DMSO-*d*_{*6*}): δ (ppm) 115.6, 116.1, 122.6, 129.6, 129.8, 132.3, 146.1, 147.6, 151.4, 151.8, 154.0, 160.1, 165.0

### 9-Benzyl-6-phenylsulfanyl-9H-purin (74)

Eine Lösung von 9-Benzyl-6-chloro-9H-purin (**29**) (423 mg, 1.73 mmol), Thiophenol (490 mg, 4.46 mmol) und Triethylamin (518 mg, 5.13 mmol) in 10 mL n-Butanol wird 4 h unter Rückfluss gerührt. Das Lösungsmittel wird abrotiert. Der ölige Rückstand wird über Nacht im Kühlschrank kristallisieren gelassen, morgens in einen Büchnertrichter überführt und mit Hexan gewaschen. Nach säulenchromatographischer Reinigung (SiO₂ 60, EtOAc/Hexan 670+330) und Trocknung unter Vakuum erhält man die Titelverbindung als gelblich weiße Substanz.
- C₁₈H₁₄N₄S (Mᵣ 318.40): 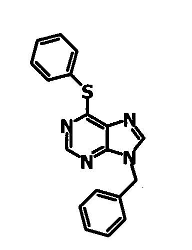
- Ausbeute: 383 mg (70%)
- Schmelzpunkt: 121 °C
- IR (ATR): 3045, 1560, 1421, 1397, 1325, 1246, 1183, 932, 854, 748, 728, 687 cm⁻¹
- ¹H-NMR (CDCl₃): δ (ppm) 5.41 (s, 2H, CH₂), 7.29-7.49 (m, 8H, Phenyl), 7.46-7.66 (m, 2H, Phenyl), 7.98 (s, 1H, C⁸-H), 8.65 (s, 1H, C²-H)
- ¹³C-NMR (CDCl₃): δ (ppm) 47.4 (CH₂), 127.2, 127.8 (2C, Phenyl), 128.6 (C⁴', Phenyl), 129.1 (2C, Phenyl), 129.2 (2C, Phenyl), 129.5 (C⁴', Phenyl), 130.6, 135.0, 135.6 (2C, Phenyl), 142.9 (C⁸), 149.0, 152.5 (C²), 160.8

### 9-Benzyl-6-(4-fluoro-phenylsulfanyl)-9H-purin (75)

Eine Lösung von 9-Benzyl-6-chloro-9H-purin (**29**) (423 mg, 1.73 mmol), 4-Fluorthiophenol (570 mg, 4.46 mmol) und Triethylamin (518 mg, 5.13 mmol) in 10 mL n-Butanol wird 4 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, EtOAc/Hexan 670+330) erhält man die Titelverbindung als gelblich weiße Substanz und Trocknung unter Vakuum.
- C₁₈H₁₃FN₄S (Mᵣ 336.39): 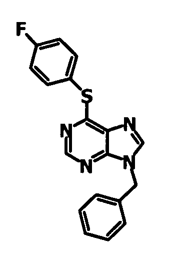
- Ausbeute: 500 mg (86%)
- Schmelzpunkt: 163 °C
- IR (ATR): 3092, 1590, 1567, 1489, 1437, 1395, 1326, 1219 (C-F), 1184, 1161, 1145, 955, 934, 825, 720, 696 cm⁻¹
- ¹H-NMR (CDCl₃): δ (ppm) 5.42 (s, 2H, CH₂), 7.12-7.37 (m, 7H, Phenyl), 7.60-7.67 (m, 2H, Phenyl), 7.98 (s, 1H, C⁸-H), 8.65 (s, 1H, C²-H)
- ¹³C-NMR (CDCl₃): δ (ppm) 47.4 (CH₂), 116.3, 116.8, 122.3, 127.8, 128.6, 129.1, 130.6, 135.0, 137.7, 137.8, 142.9 (C⁸), 149.0, 152.4 (C²), 160.6, 161.1, 166.1

### 9-(4-Fluoro-benzyl)-6-phenylsulfanyl-9H-purin (76)

Eine Lösung von 6-Chloro-9-(4-fluoro-benzyl)-9H-purin (**31**) (453 mg, 1.73 mmol), Thiophenol (490 mg, 4.46 mmol) und Triethylamin (518 mg, 5.13 mmol) in 10 mL n-Butanol wird 4 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, EtOAc/Hexan 670+330) und Trocknung unter Vakuum erhält man die Titelverbindung als gelblich weiße Substanz.
- C₁₈H₁₃FN₄S (Mᵣ 336.39): 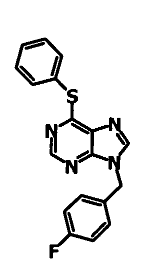
- Ausbeute: 505 mg (87%)
- Schmelzpunkt: 128 °C
- IR (ATR): 3090, 1567, 1509, 1390, 1335, 1218 (C-F), 1185, 1156, 935, 856, 747, 689 cm⁻¹
- ¹H-NMR (CDCl₃): δ (ppm) 5.38 (s, 2H, CH₂), 6.99-7.08 (m, 2H, Phenyl), 7.26-7.33 (m, 2H, Phenyl), 7.45-7.49 (m, 3H, Phenyl), 7.64-7.69 (m, 2H, Phenyl), 7.97 (s, 1H, C⁸-H), 8.64 (s, 1H, C²-H)
- ¹³C-NMR (CDCl₃): δ (ppm) 46.7 (CH₂), 115.9, 116.3, 127.1, 129.3, 129.5, 129.6, 129.8, 130.6, 131.0, 135.6, 142.6 (C⁸), 148.9, 152.5 (C²), 160.2, 160.9, 165.2

### 9-(4-Fluoro-benzyl)-6-(4-fluoro-phenylsulfanyl)-9H-purin (77)

Eine Lösung von 6-Chloro-9-(4-fluoro-benzyl)-9H-purin (**31**) (453 mg, 1.73 mmol), 4-Fluorthiophenol (570 mg, 4.46 mmol) und Triethylamin (518 mg, 5.13 mmol) in 10 mL n-Butanol wird 4 h unter Rückfluss gerührt. Das Lösungsmittel wird abrotiert. Der Rückstand wird in einen Büchnertrichter überführt und mit Hexan gewaschen. Nach säulenchromatographischer Reinigung (SiO₂ 60, EtOAc/Hexan 670+330) und Trocknung unter Vakuum erhält man die Titelverbindung als gelblich weiße Substanz.
- C₁₈H₁₂F₂N₄S (Mᵣ 354.38): 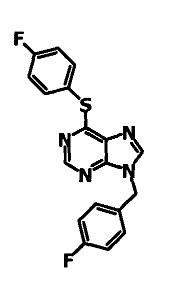
- Ausbeute: 480 mg (78%)
- Schmelzpunkt: 188°C
- IR (ATR): 3092, 1590, 1567, 1490, 1395, 1326, 1222 (C-F), 1182, 1162, 1094, 931, 856, 826, 769, 755 cm⁻¹
- ¹H-NMR (CDCl₃): δ (ppm) 5.39 (s, 2H, CH₂), 7.00-7.33 (m, 6H, Phenyl), 7.60-7.67 (m, 2H, Phenyl), 7.98 (s, 1H, C⁸-H), 8.64 (s, 1H, C²-H)
- ¹³C-NMR (CDCl₃): δ (ppm) 46.7 (CH₂), 115.9, 116.3, 116.8, 122.2, 129.6, 129.8, 130.6, 130.8, 137.7, 137.8, 142.7 (C⁸), 148.9, 152.5 (C²), 160.3, 160.7, 161.2, 165.2, 166.1

### 8-Bromo-9-methyl-6-phenylsulfanyl-9H-purin (78)

Zu einer auf -78 °C abgekühlten Lösung von Diisopropylamin (1.41 g, 14 mmol) in THF abs. (30 mL) in einem ausgeheizten und mit Argon gespülten DHK wird innerhalb von 15 min *n*-BuLi (15 %ige Lösung in *n*-Hexan, 6 g, 14.1 mmol) zugetropft. Nach beendeter Zugabe wird 1 h bei -78 °C gerührt. Innerhalb von 20 min wird tropfenweise eine Lösung von 9-Methyl-6-phenylsulfanyl-9H-purin (17) (2.42 g, 10 mmol) in THF abs. (20 mL) zugegeben und die Lösung nach beendeter Zugabe 1 h bei -78 °C gerührt. Innerhalb von 20 min wird eine Lösung von 1,2-Dibromtetrachlorethan (6.5 g, 20 mmol) in THF abs. (20 mL) zugetropft und die Lösung nach beendeter Zugabe 1 h bei -78 °C gerührt. Es wird mit gesättigter NH₄Cl-Lösung (10 mL) versetzt, auf Raumtemperatur erwärmt und das THF abrotiert. Zur zurückbleibenden wässrigen Phase werden weitere 50 mL Wasser gegeben und es wird mit je 50 mL Dichlormethan dreimal extrahiert. Die vereinigten Dichlormethanphasen werden über Na₂SO₄ getrocknet und einrotiert. Der Rückstand wird mit 100 mL Hexan kurz unter Rückfluss gerührt und heiß filtriert. Das Filtrat wird verworfen und der Rückstand säulenchromatographisch gereinigt (SiO₂ 60, CH₂Cl₂/EtOH 9.5+0.5).
- C₁₂H₉BrN₄S (Mᵣ 321.20): 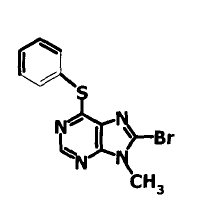
- Ausbeute: 1.76 g (55%)
- Schmelzpunkt: 166 °C
- IR (ATR): 3068, 1564, 1473, 1429, 1319, 1236, 1150, 1064, 940 859, 749, 688 cm⁻¹
- ¹H-NMR (CDCl₃): δ (ppm) 3.82 (s, 3H, N⁹-CH₃), 7.44-7.67 (m, 5H, Phenyl), 8.56 (s, 1H, C²-H)
- ¹³C-NMR (CDCl₃): δ (ppm) 30.6 (N⁹-CH₃), 126.9, 129.3 (2C, Phenyl), 129.6 (C⁴', Phenyl), 130.9, 132.0, 135.6 (2C, Phenyl), 150.2, 152.2 (C²), 159.3

### 4-(9-Methyl-6-phenylsulfanyl-9H-purin-8-yl)-but-3-yn-1-ol (79)

8-Bromo-9-methyl-6-phenylsulfanyl-9H-purin (**78**) (482 mg, 1.5 mmol), But-3-yn-1-ol (158 mg, 2.25 mmol), Triethylamin (505 mg, 5 mmol), PdCl₂(PPh₃)₂ (81 mg, 0.12 mmol) und Cul (40 mg, 0.23 mmol), werden in einem 50 mL Rundkolben vorgelegt. Als Lösungsmittel wird Dichlormethan (10 mL) hinzugefügt und unter Argonatmosphäre und Lichtschutz 7 h refluxiert. Die ungelösten Anteile werden abfiltriert und das Filtrat einrotiert. Der braune Rückstand wird säulenchromatographisch gereinigt (SiO₂ 60, CH₂Cl₂/EtOH/EtOAc 9+2+1).
- C₁₆H₁₄N₄OS (Mᵣ 310.38): 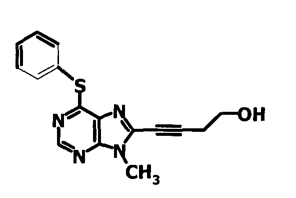
- Ausbeute: 30 mg (6.5%)
- Schmelzpunkt: 231 °C (Zersetzung)
- IR (ATR): 3307, 2244 (C≡C), 1565, 1469, 1433, 1334, 1246, 1155, 1056 948, 865, 747, 689 cm⁻¹
- ¹H-NMR (DMSO-d6): δ (ppm) 2.76 (t, 2H, CH₂, ³J(H,H)=6.2 Hz), 3.69(q, 2H, CH₂, ³J(H,H)=6.2 Hz), 3.80 (s, 3H, N⁹-CH₃), 5.10 (t, 1H, OH, ³J(H,H)=6.2 Hz), 7.51-7.64 (m, 5H, Phenyl), 8.58 (s, 1H, C²-H)
- ¹³C-NMR (DMSO-d6): δ (ppm) 23.6 (C², Butynol), 29.9 (N⁹-CH₃), 59.3 (C¹, Butynol), 70.7 (C⁴, Butynol), 98.9 (C³, Butynol), 126.9, 129.7 (2C, Phenyl), 129.9 (C⁴', Phenyl), 135.1, 135.8 (2C, Phenyl), 138.4, 149.2, 152.5 (C²), 158.5

## Patentansprüche

1. Verwendung einer Verbindung der Formel I 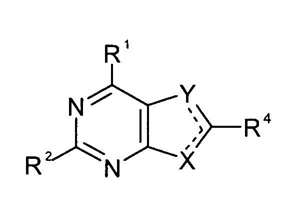 worin
R¹ für H, Phenylamino, Halogen-substituiertes Phenylamino, Phenoxy, Halogen-substituiertes Phenoxy, Benzylsulfanyl, am Phenylring Halogen-substituiertes Benzylsulfanyl, Phenylsulfanyl, Halogen-substituiertes Phenylsulfanyl, Phenyl, Halogen-substituiertes Phenyl, Styryl, am Phenylring Halogen-substituiertes Styryl, Benzyl, am Phenylring Halogen-substituiertes Benzyl, Amino, C₁-C₄-Alkoxyl, Halogen oder H steht;
R² für H oder C₁-C₄-Alkyl steht,
die gestrichelte Linie eine chemische Bindung zwischen X und CR⁴ oder zwischen Y und CR⁴ symbolisiert;
X für N oder NR³ steht und Y für N, CR⁵ oder NR⁶ steht, wobei X für NR³ und Y für N oder CR⁵ stehen, wenn die gestrichelte Linie eine Bindung zwischen Y und CR⁴ bedeutet oder X für N und Y für NR⁶ stehen, wenn die gestrichelte Linie eine Bindung zwischen X und CR⁴ bedeutet;
R³ für H, C₁-C₄-Alkyl, Benzyl, am Phenylring Halogen-substituiertes Benzyl, Phenyl oder Halogen-substituiertes Phenyl steht;
R⁴ für H, Halogen, Phenyl, das gegebenenfalls durch 1 oder 2 C₁-C₄-Alkyl-S(O)ₙ-substituiert ist, oder für -C≡C-(CH₂)ₘ-OH steht, wobei n für 0, 1 oder 2 steht und m für 1, 2 oder 3 steht;
R⁵ für H steht und
R⁶ für H, Phenyl, Halogen-substituiertes Phenyl, Benzyl oder am Phenylring Halogen-substituiertes Benzyl steht
oder eines physiologisch verträglichen Salzes dieser Verbindungen
zur Herstellung eines pharmazeutischen Mittels zur selektiven Inhibierung der Aktivität von Kinasen.

2. Verwendung nach Anspruch 1 von Verbindungen der Formel I, worin R¹ für Phenylamino, Halogen-substituiertes Phenylamino, Benzylsulfanyl, am Phenylring Halogen-substituiertes Benzylsulfanyl, Phenylsulfanyl, Halogen-substituiertes Phenylsulfanyl, Phenyl, Halogen-substituiertes Phenyl, Amino, C₁-C₄-Alkoxyl, Styryl oder am Phenylring Halogen-substituiertes Styryl steht.

3. Verwendung nach Anspruch 1 oder 2 zur Hemmung einer Kinase, die ausgewählt ist unter c-RAF, GSK3β, JNK3, Lck, MAPK1, MAPK2, MEK1, PKA, PKB, PKC, ROCK-II, SAPK2a

4. Verwendung nach Anspruch 3, wobei die Kinase ausgewählt ist unter c-RAF, Lck, MAPK1, MAPK2 und SAPK2a.

5. Verwendung nach einem der vorhergehenden Ansprüche zur Hemmung des Tumorwachstums, zur Behandlung von neurodegenerativen Krankheiten, Diabetes mellitus, Schlaganfall, Krebs, chronisch entzündlichen Erkrankungen, rheumatoider Arthritis, Asthma, chronischen entzündlichen Darmerkrankungen, Psoriasis, Multipler Sklerose, erektiler Dysfunktion, Tumorerkrankungen, die erhöhte Proteinkinase-B-Aktivität aufweisen, neuronaler Störungen, kardiovaskulärer Erkrankungen und diabetischer Retinopathie.

6. Verwendung nach Anspruch 1 einer Verbindung der Formel I, worin
R¹ für Benzylsulfanyl, am Phenylring Halogen-substituiertes Benzylsulfanyl, Phenylamino, Halogen-substituiertes Phenylamino, Phenylsulfanyl, Halogen-substituiertes Phenylsulfanyl, Styryl, am Phenylring Halogen-substituiertes Styryl oder Amino steht;
R² für H oder C₁-C₄-Alkyl steht;
die gestrichelte Linie eine Bindung zwischen X und CR⁴ oder zwischen Y und CR⁴ bedeutet;
X für N und Y für CR⁵ oder NR⁶ stehen, wenn die gestrichelte Linie eine Bindung zwischen X und CR⁴ bedeutet oder
X für NR³ und Y für N stehen, wenn die gestrichelte Linie eine Bindung zwischen Y und CR⁴ bedeutet;
R³ für H, C₁-C₄-Alkyl, Benzyl oder am Phenylring Halogen-substituiertes Benzyl steht;
R⁴ und R⁵ für H stehen; und
R⁶ für H, Benzyl oder am Phenylring Halogen-substituiertes Benzyl steht, oder eines physiologisch verträglichen Salzes davon zur Hemmung des Tumorwachstums.

7. Verwendung nach Anspruch 1 einer Verbindung der Formel la 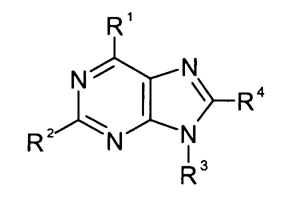 worin R¹ für Benzylsulfanyl, am Phenylring Halogen-substituiertes Benzylsulfanyl, Phenylamino, Halogen-substituiertes Phenylamino, Phenyl oder Halogen-substituiertes Phenyl steht,
R² für H steht,
R³ für H oder C₁-C₄-Alkyl steht, und
R⁴ für H, Phenyl oder Halogen-substituiertes Phenyl steht oder eines physiologisch verträglichen Salzes davon, zur Behandlung von neurodegenerativen Erkrankungen, Diabetes mellitus, Schlaganfall, Krebs und chronischen entzündlichen Erkrankungen.

8. Verwendung nach Anspruch 1 einer Verbindung der Formel Id 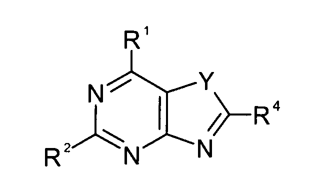 worin R¹ für C₁-C₄-Alkoxy oder Amino steht, R² und R⁴ für H stehen, Y für NR⁶ steht, wobei R⁶ für Benzyl oder am Phenylring Halogen-substituiertes Benzyl steht oder eines physiologisch verträglichen Salzes davon zur Behandlung von neurodegenerativen und chronischen entzündlichen Erkrankungen.

9. Verwendung nach Anspruch 1 einer Verbindung der Formel Formel le 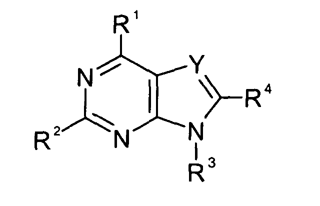 worin R¹ für Phenylamino, Halogen-substituiertes Phenylamino, Phenylsulfanyl, Halogen-substituiertes Phenylsulfanyl, Styryl oder am Phenylring Halogen-substituiertes Styryl steht,
R² für H steht,
R³ für H oder C₁-C₄-Alkyl steht,
R⁴ für H oder Phenyl, das gegebenenfalls durch ein oder zwei C₁-C₄-Alkyl-S(O)ₙ-substituiert ist, steht, wobei n für 0, 1 oder 2 steht,
Y für N oder CR⁵ steht und
R⁵ für H steht oder eines physiologisch verträglichen Salzes davon zur Behandlung von rheumatoider Arthritis, Asthma, chronischer entzündlicher Darmerkrankung, Psoriasis oder Multipler Sklerose.

10. Verwendung nach Anspruch 1 einer Verbindung der Formel la 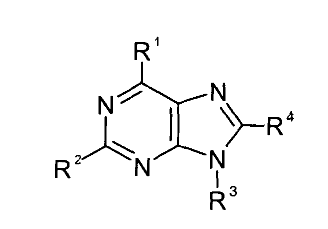 worin R¹ für Phenylamino, Halogen-substituiertes Phenylamino, Styryl oder Halogen-substituiertes Styryl steht,
R² und R³ für H oder C₁-C₄-Alkyl stehen und
R⁴ für H steht,
oder Verbindungen der Formel Ib, worin R¹ für Amino steht, R² und R⁴ für H stehen und R⁶ für Benzyl oder am Phenylring Halogen-substituiertes Benzyl steht
oder
worin R¹ für Phenylamino oder Halogen-substituiertes Phenylamino steht, R² für H oder C₁-C₄-Alkyl steht und
R³ und R⁴ für H stehen oder eines physiologisch verträglichen Salzes davon zur Behandlung entzündlicher Erkrankungen.

11. Verwendung nach Anspruch 1 einer Verbindung der Formel le 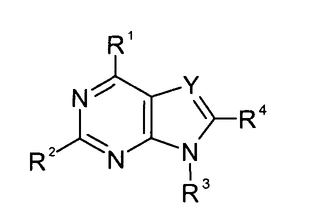 worin R¹ für Phenylamino, Halogen-substituiertes Phenylamino, Phenylsulfanyl oder Halogen-substituiertes Phenylsulfanyl steht,
R², R³ und R⁴ für H stehen,
Y für N oder CR⁵ steht und
R⁵ für H steht oder eines physiologisch verträglichen Salzes davon zur Behandlung entzündlicher Erkrankungen.

12. Verwendung nach Anspruch 1 einer Verbindung der Formel la 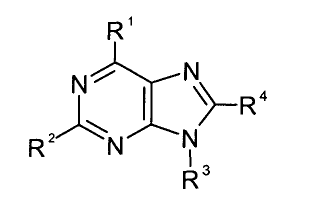 worin R¹ für Phenylsulfanyl, Halogen-substituiertes Phenylsulfanyl, Phenyl oder Halogen-substituiertes Phenyl steht,
R² für H steht,
R³ für H oder C₁-C₄-Alkyl steht,
R⁴ für Phenyl oder -C≡C-(CH₂)ₘ-OH steht, wobei m für 1, 2 oder 3 steht
oder worin R¹ für Phenylamino oder Halogen-substituiertes Phenylamino steht und R², R³ und R⁴ für H stehen,
oder eines physiologisch verträglichen Salzes davon zur Behandlung von Krebs.

13. Verwendung nach Anspruch 1 einer Verbindung der Formel le 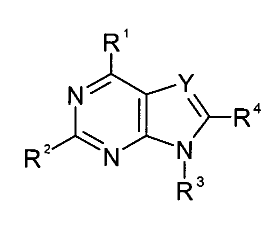 worin R¹ für Phenylamino, Halogen-substituiertes Phenylamino, Phenylsulfanyl oder Halogen-substituiertes Phenylsulfanyl steht,
R² und R⁴ für H stehen,
R³ für H oder C₁-C₄-Alkyl steht,
Y für N oder CR⁵ steht und
R⁵ für H steht oder eines physiologisch verträglichen Salzes davon zur Behandlung erektiler Dysfunktion.

14. Verwendung nach Anspruch 1 einer Verbindung der Formel le 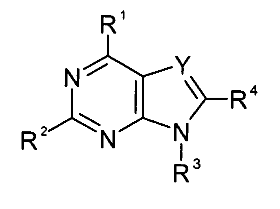 worin R¹ für Phenylamino, Halogen-substituiertes Phenylamino, Benzylsulfanyl, am Phenylring Halogen-substituiertes Benzylsulfanyl, Phenylsulfanyl oder Halogen-substituiertes Phenylsulfanyl steht,
R² für H steht,
R³ für H, C₁-C₄-Alkyl, Benzyl oder am Phenylring Halogen-substituiertes Benzyl steht,
R⁴ für H, Phenyl oder Phenyl, das durch C₁-C₄-Alkyl-S(O)ₙ- substituiert ist, steht, wobei n für 0,1 oder 2 steht,
Y für N oder CR⁵ steht und
R⁵ für H steht oder eines physiologisch verträglichen Salzes davon zur Behandlung entzündlicher Erkrankungen.

15. Verwendung nach Anspruch 1 einer Verbindung der Formel I 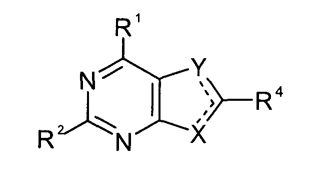 worin R¹ für Benzylsulfanyl, am Phenylring Halogen-substituiertes Benzylsulfanyl, Phenylsulfanyl oder Halogen-substituiertes Phenylsulfanyl steht,
R² für H oder C₁-C₄-Alkyl steht,
die gestrichelte Linie eine chemische Bindung zwischen X und CR⁴ oder zwischen Y und CR⁴ bedeutet,
X für N oder NR³ steht und Y für N, CR⁵ oder NR⁶ steht, wobei X für NR³ und
Y für N oder CR⁵ steht, wenn die gestrichelte Linie eine Bindung zwischen Y und CR⁴ bedeutet oder X für N und Y für NR⁶ steht, wenn die gestrichelte Linie eine chemische Bindung zwischen X und CR⁴ bedeutet,
R³ für H, C₁-C₄-Alkyl, Benzyl oder am Phenylring Halogen-substituiertes Benzyl steht,
R⁴ für H, Phenyl oder Phenyl, das durch 1 oder 2 C₁-C₄-Alkyl-S(O)ₙsubstituiert ist, oder für -C≡C-(CH₂)ₘ-OH steht,
n für 0, 1 oder 2 steht,
m für 1, 2 oder 3 steht,
R⁵ für H, und
R⁶ für H, Phenyl, Halogen-substituiertes Phenyl oder am Phenylring Halogen-substituiertes Benzyl steht,
und die physiologisch verträglichen Salze davon.

16. Pharmazeutisches Mittel, enthaltend wenigstens eine Verbindung nach Anspruch 15 zusammen mit pharmazeutisch annehmbaren Hilfsstoffen.
